# EUROPÄISCHE PATENTSCHRIFT

(11) **EP 0 927 174 B1**
(45) Veröffentlichungstag und Bekanntmachung des Hinweises auf die Patenterteilung: **12.12.2007**
(21) Anmeldenummer: 97942012.2
(22) Anmeldetag: 10.09.1997
(51) Int. Cl.: C07D 295/12, C07D 211/58, C07D 211/26, C07C 219/06, C07C 219/08, C07C 215/14, A61K 31/23, A61K 31/495, C07D 401/04

(54) **NEUE AMINOALKOHOLDERIVATE, VERFAHREN ZU DEREN HERSTELLUNG UND DIESE VERBINDUNGEN ENTHALTENDE ARZNEIMITTEL UND REAGENZIEN**
NEW AMINO ALCOHOL DERIVATIVES, PROCESS FOR THE PRODUCTION THEREOF AND MEDICAMENTS AND REAGENTS CONTAINING THESE COMPOUNDS
NOUVEAUX DERIVES D'AMINOALCOOLS, LEUR PROCEDE DE PRODUCTION, ET MEDICAMENTS ET REACTIFS CONTENANT CES COMPOSES

(30) Priorität: 12.09.1996 DE 19637043
(43) Veröffentlichungstag der Anmeldung: 07.07.1999
(73) Patentinhaber: MediGene AG, 82152 Planegg/Martinsried (DE)
(72) Erfinder: FRIEBE, Walter-Gunar, D-68165 Mannheim (DE); DIMOUDIS, Nikolaos, D-82407 Wielenbach (DE); MICHAELIS, Uwe, D-82362 Weilheim (DE); KNIPP, Bernhard, D-51515 Kürten (DE)
(74) Vertreter: Weickmann, Heinrich
(86) Internationale Anmeldenummer: PCT/EP1997/004944
(87) Internationale Veröffentlichungsnummer: WO 1998/011082

(56) Entgegenhaltungen:
- EP-A- 0 002 901
- WO-A-94/05624
- WO-A-95/25542
- WO-A-96/17823
- WO-A-97/00241
- JP-A- 5 232 660
- JP-A- 63 025 654
- US-A- 3 689 494
- US-A- 5 491 263
- US-A- 5 635 487
- PATENT ABSTRACTS OF JAPAN vol. 095, no. 003, 28.April 1995 & JP 06 340598 A (LION CORP.), 13.Dezember 1994,
- PATENT ABSTRACTS OF JAPAN vol. 018, no. 163 (C-1181), 18.März 1994 & JP 05 331118 A (KAO CORP.), 14.Dezember 1993,
- CAS REGISTRY HANDBOOK 1965-1971: , AMERICAN CHEMICAL SOCIETY XP002051753 * CN: 5505-42-0 *
- CAS REGISTRY HANDBOOK 1974 SUPPLEMENT: , AMERICAN CHEMICAL SOCIETY XP002051754 * CN: 45321-99-1 *
- CHEMICAL ABSTRACTS, vol. 71, no. 7, 18.August 1969 Columbus, Ohio, US; abstract no. 30380g, XP002051755 & J.H. AGER ET AL.: JOURNAL OF PHARMACEUTICAL SCIENCES., Bd. 58, Nr. 4, 1969, WASHINGTON US, Seiten 499-500,
- CHEMICAL ABSTRACTS, vol. 100, no. 1, 2.Januar 1984 Columbus, Ohio, US; abstract no. 6612f, XP002051756 & P. HEGYES ET AL.: JOURNAL OF ORGANOMETALLIC CHEMISTRY., Bd. 251, Nr. 3, 1983, LAUSANNE CH, Seiten 289-294, in der Anmeldung erwähnt
- CHEMICAL ABSTRACTS, vol. 78, no. 25, 25.Juni 1973 Columbus, Ohio, US; abstract no. 159552p, XP002051757 & SV. ZIKOLOVA ET AL.: TR. NAUCHNOIZSLED. KHIM.-FARM. INST., Bd. 7, 1972, Seiten 79-90,

## Beschreibung

Die vorliegende Erfindung betrifft neue Aminoalkoholderivate, Verfahren zu deren Herstellung sowie Arzneimittel und Reagenzien, die diese Substanzen enthalten.

Gegenstand der Erfindung sind Arzneimittel der allgemeinen Formel I 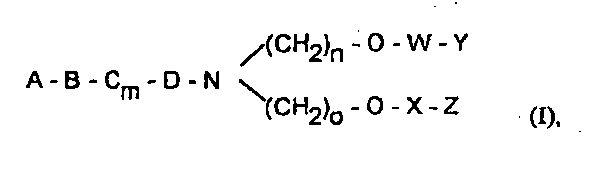 in welcher
- A: eine Gruppe NR₁R₂, eine Gruppe NR₁(CH₂)ₚNR₃R₄, eine Gruppe (C=NH)NH₂ oder einen Pyridinylrest,
- B und D: gleich oder verschieden jeweils eine Bindung, einen C₁- bis C₆-Alkylenrest oder eine Gruppe NR₅-C₂ bis C₆-Alkylen.
- C: Piperidindiyl oder Piperazindiyl,
- W und X: gleich oder verschieden jeweils eine Bindung oder eine Carbonylgruppe.
- Y und Z: gleich oder verschieden jeweils einen gesättigten oder ungesättigten Kohlenwasserstoffrest mit 7 bis 24 Kohlenstoffatomen,
- R₁ bis R₅: gleich oder verschieden jeweils Wasserstoff oder einen C₁- bis C₆-Alkylrest,
- m: eine ganze Zahl 0, 1 oder 2, wobei für m gleich 2 die beiden Reste C gleich oder verschieden sein können,
- n und o: gleich oder verschieden jeweils ganze Zahlen 2, 3 oder 4 und
- p: eine ganze Zahl von 2 bis 6
bedeuten
sowie deren physiologisch verträgliche Salze,
mit der Maßgabe, daß keine Hydrazinderivate umfaßt sind und daß m nicht 0 sein kann, wenn A eine Gruppe (C=NH)NH₂ bedeutet und B und D gleich oder verschieden eine Bindung oder einen Alkylenrest darstellen.

Die in den Bedeutungen von B, D, Y, Z und R₁ bis R₅ enthaltenen Alkyl-, Alkylen- und Kohlenwassertoffreste können geradkettig oder verzweigt sein. Unter Pyridinyl-Rest wird eine unsubstituiertes oder mit geradkettigem oder verzweigtem C₁-C₆ gegebenenfalls mehrfach substituiertes Pyridin verstanden.

Bevorzugt sind für m die Werte 1 oder 2. Ebenso bevorzugt sind Verbindungen der allgemeinen Formel I, die mehr als 2 Stickstoffe enthalten oder für den Fall, daß A (C=NH)NH₂ entspricht, solche die mehr wie 3 Stickstoffe enthalten.

Gegenstand der Erfindung sind außerdem neue Aminoalkoholderivate der allgemeinen Formel I. 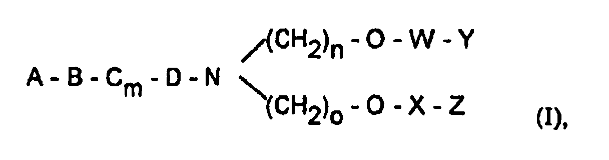 in welcher
- A: eine Gruppe NR₁R₂, eine Gruppe NR₁(CH₂)ₚNR₂R₄, eine Gruppe (C=NH)NH₂ oder einen Pyridinylrest,
- B und D: gleich oder verschieden jeweils eine Bindung, einen C₁- bis C₆-Alkylenrest oder eine Gruppe NR₅-C₂- bis C₆-Alkylen,
- C: Piperidindiyl oder Piperazindiyl,
- W und X: gleich oder verschieden jeweils eine Bindung oder eine Carbonylgruppe.
- Y und Z: gleich oder verschieden jeweils einen gesättigten oder ungesättigten Kohlenwasserstoffrest mit 7 bis 24 Kohlenstoffatomen,
- R₁ bis R₅: gleich oder verschieden jeweils Wasserstoff oder einen C₁- bis C₆-Alkylrest,
- m: eine ganze Zahl 1 oder 2, wobei für m gleich 2 die beiden Reste C gleich oder verschieden sein können,
- n und o: gleich oder verschieden jeweils ganze Zahlen 2, 3 oder 4 und

- p: eine ganze Zahl von 2 bis 6
bedeuten
sowie deren physiologisch verträgliche Salze,
mit der Maßgabe, daß keine Hydrazinderivate umfaßt sind :

Im Sinne der vorliegenden Erfindung sind die folgenden Bedeutungen in Verbindungen der Formel I unabhängig voneinander bevorzugt. Dies gilt sowohl für die Verbindungen wie auch für die Medikamente, die solche Verbindungen enthalten und ebenso auch für die entsprechenden therapeutischen Verwendungen dieser Verbindungen.
- A: bedeutet NH₂ oder N(CH₃)₂ oder
- B und D: sind gleich oder verschieden und bedeuten eine Bindung, einen C₁-C₃-Alkylenrest oder bei m=0, im Falle des Medikaments, N(CH₃)C₃bisC₄-Alkylenrest oder
- C: bedeutet Piperidindiyl oder
- m: bedeutet 0, im Falle des Medikaments, oder 1 oder
- W und X: bedeuten jeweils CO oder
- Y: bedeutet C₁₃H₂₇ oder C₁₇H₃₃ oder
- Z: bedeutet C₁₃H₂₇ oder C₁₇H₃₃ oder
- n und o: bedeuten jeweils 2.

Ganz besonders bevorzugt sind Verbindungen, die alle oben genannten Bedeutungen gleichzeitig erfüllen.

Die Verbindungen der Formel I weisen wertvolle pharmakologische Eigenschaften auf, insbesondere können sie die Einschleusung von biologisch aktiven Molekülen in prokaryotische oder eukaryotische Zellen erleichtern. Sie eignen sich daher besonders zur Einbringung von Proteinen, Nukleinsäuren, wie z.B. DNA, cDNA, mRNA, PNA, Antisense-Polynucleotiden und therapeutisch wirksamen niedermolekularen Verbindungen wie beispielsweise Peptidhormone Cytostatika und Antibiotika in Zielzellen innerhalb oder außerhalb des Organismus. Die neuen erfindungsgemäßen Verbindungen sind damit besonders geeignet zur effizienten gentherapeutischen Behandlung von Säugern, bevorzugt von humanen Patienten. Diese Verbindungen eignen sich auch zur Herstellung von Arzneimittelkombinationen in Krebstherapie, antiviraler Therapie, Infektionstherapie und bei Krankheiten verursacht durch Disregulation. Im Gegensatz zu viralen Carriern für Genkonstrukte zeigen nichtvirale Genfähren oft nur geringe Immunogenität. Die Effizienz und Persistenz der mit nichtviralen Genfähren vermittelten Genexpression ist jedoch bisher nicht befriedigend. Außer einer Verbesserung der Genexpression zeigen die Verbindungen der allgemeinen Formel I den Vorteil, daß sie relativ leicht abbaubar sind wegen der C₂-C₄-Alkyl-O-Ketten am tertiären Stickstoff N der Formel I.

Außer den in den Beispielen aufgeführten Verbindungen sind Gegenstand der Erfindung insbesondere alle Substanzen, die jede mögliche Kombination der in den Beispielen genannten Bedeutungen der Variablen aufweisen.

Das erfindungsgemäße Verfahren zur Herstellung der Verbindungen der Formel I ist dadurch gekennzeichnet, daß man in an sich bekannter Weise eine Verbindung der allgemeinen Formel II 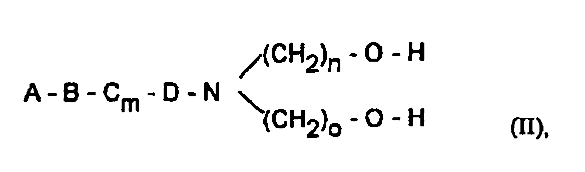 in welcher A, B, C, D, n und o die oben genannte Bedeutung haben und m=1 ! oder 2 ist, mit einer Verbindung der allgemeinen Formel III und einer Verbindung der allgemeinen Formel IV

E-W-Y (III)

G-X-Z (IV),

in weicher W, X, Y und Z die obengenannte Bedeutung haben und E und G reaktive Reste darstellen,
umsetzt und anschließend gewünschtenfalls eine in A, B oder D enthaltene Schutzgruppe abspaltet, ein für A stehendes Wasserstoffatom in eine Gruppe (C=NH)NH₂ überfiihrt, eine als Säureadditionssalz vorliegende Verbindung in die freie Base umwandelt oder eine als Base vorliegende Verbindung durch Neutralisation mit einer nichttoxischen Säure in ein physiologisch verträgliches Salz überführt.

Reaktive Reste E und G sind nucleofuge Gruppen wie beispielsweise Halogenatome, Sulfonat- oder Sulfatgruppen oder Säurereste von aktivierten Estern, Anhydriden oder gemischten Anhydriden.

Die Umsetzung von Verbindungen der Formel II mit Verbindungen der Formeln III und IV erfolgt zweckmäßig in einem inerten Lösungsmittel wie einem Ether, beispielsweise Tetrahydrofuran, oder einem Amid wie Dimethylformamid, oder in Pyridin, gewünschtenfalls in Gegenwart einer Base wie Triethylamin oder Ethyldiisopropylamin oder einem Alkalialkoholat, jedoch können auch die Reagenzien der allgemeinen Formel III oder IV unverdünnt eingesetzt oder, für den Fall, daß W und X für eine Carbonylgruppe stehen, auch eine Säure wie Essigsäure oder Trifluoressigsäure als Lösungsmittel verwandt werden.

Eine Abspaltung einer in A, B oder D enthaltenen Schutzgruppe erfolgt abhängig von der chemischen Eigenart dieser Gruppe, beispielsweise durch saure oder basische Hydrolyse oder hydrogenolytisch. Eine sauer abspaltbare Schutzgruppe ist beispielsweise der tert.-Butoxycarbonylrest.
Eine Umwandlung eines für A stehenden Wasserstoffatoms in eine Amidinogruppe kann beispielsweise durch Umsetzung mit Cyanamid oder Pyrazol-1-carboxamidin erfolgen.

Die Ausgangsverbindungen der allgemeinen Formel II sind überwiegend neu (insbesondere wenn m für 1 oder 2 steht) und ebenfalls Gegenstand der Erfindung.

Sie können aus bekannten Ausgangstoffen nach literaturbekannten Methoden hergestellt werden.

Als pharmakologisch verträgliche Salze kommen insbesondere Salze mit nichttoxischen anorganischen oder organischen Säuren wie beispielsweise Salzsäure, Schwefelsäure, Phosphorsäure, Bromwasserstoffsäure, Essigsäure, Trifluoressigsäure, Milchsäure, Zitronensäure, Äpfelsäure, Benzoesäure, Salicylsäure, Malonsäure, Maleinsäure, Bernsteinsäure oder Diaminocapronsäure in Frage.

Die Salze erhält man in üblicher Weise beispielsweise durch Neutralisation der Verbindungen der Formel I mit den entsprechenden Säuren.

Zur Herstellung von Arzneimitteln oder Transferreagentien werden die erfindungsgemäßen Verbindungen einzeln oder als Kombination, gewünschtenfalls unter Verwendung von Co-Lipiden, mit einem biologisch aktiven Molekül, beispielsweise einem Polynucleotid, in einem geeigneten Verhältnis kombiniert und in flüssiger, vorzugsweise wässriger, oder fester, vorzugsweise lyophilisierter Form in vivo oder in vitro appliziert. Die in-vivo-Applikation kann oral, parenteral, topisch, transmucosal oder durch Einbringung in eine Körperhöhle des Patienten erfolgen. Ebenso ist eine verzögerte Freisetzung aus einer biologisch abbaubaren Matrix oder eine Verabreichung als Aerosol oder inhalierbare Pulverapplikation möglich.

Die verabreichte Dosis hängt vom Alter, der Gesundheit und dem Gewicht des Empfangers, dem Ausmaß der Krankheit, der Art gleichzeitiger gegebenfalls durchgefuhrter weiterer Behandlungen, der Häufigkeit der Behandlungen und der Art der gewünschten Wirkung ab und kann vom Fachmann experimentell ermittelt werden.

Außer den in den Beispielen genannten Substanzen sind im Sinne der Erfindung die folgenden Verbindungen bevorzugt:
1. Ölsäure-2-{(2-oleoyloxy-ethyl)-[3-(3,4,5,6-tetrahydro-2*H*-[1,4']bipyridinyl-4-ylamino)-propyl]-amino} -ethylester
2. Dodecansäure-2-[(3-[(3-dimethylamino-propyl)-methyl-amino]-propyl}-(2-dodecanoyloxy-ethyl}-amino]-ethylester
3. Tetradecansäure-2-[{3-[(3-dimethylamino-propyl)-methyl-amino]-propyl}-(2-tetradecanoyloxy-ethyl)-amino]-ethylester
4. Dodecansäure-2-[{3-[(3-diethylamino-propyl)-methyl-amino]-propyl}-(2-dodecanoyloxy-ethyl)-amino]-ethylester
5. Tetradecansäure-2-[{3-[(3-diethylamino-propyl)-methyl-amino]-propyl}-(2-tetradecanoyloxy-ethyl)-amino]-ethylester
6. Dodecansäure-2-{[3-(4-amino-butylamino)-propyl]-(2-dodecanoyloxy-ethyl)-amino}-ethylester
7. Dodecansäure-2-{(2-dodecanoyloxy-ethyl)-[1-(2-amino-ethyl)-piperidin-4-yl]-amino -ethylester
8. Ölsäure-2-{(2-oleoyloxy-ethyl)-[1-(2-amino-ethyl)-piperidin-4-yl]-amino}-ethylester
9. Dodecansäure-2-{(2-dodecanoyloxy-ethyl)-[1-(2-amino-ethyl)-piperidin-4-yl-methyl]-amino}-ethylester
10. Ölsäure-2-{(2-oleoyloxy-ethyl)-[1-(2-amino-ethyl)-piperidin-4-yl-methyl]-amino} -ethylester
11. Dodecansäure-2-{[2-(4-amino-piperidin-1-yl)-ethyl]-(2-dodecanoyloxy-ethyl)-amino}-ethylester
12. Dodecansäure-2-{[4-(4-amino-piperidin-1-yl)-butyl]-(2-dodecanoyloxy-ethyl)-amino}-ethylester
13. Tetradecansäure-2-{[2-(4-amino-piperidin-1-yl)-ethyl]-(2-tetradecanoyloxy-ethyl)-amino}-ethylester
14. Tetradecansäure-2-{[4-(4-amino-piperidin-1-yl)-butyl]-(2-tetradecanoyloxy-ethyl)-amino}-ethylester
15. Ölsäure-2-{[2-(4-amino-piperidin-1-yl)-ethyl]-(2-oleoyloxy-ethyl)-amino}-ethylester
16. Ölsäure-2-{[4-(4-amino-piperidin-1-yl)-butyl]-(2-oleoyloxy-ethyl)-amino}-ethylester
17. Ölsäure-2-{(2-oleoyloxy-ethyl)-[1-(2-amino-ethyl)-piperidin-4-yl]-amino}-ethylester
18. Ölsaure-2-{(2-oleoyloxy-ethyl)-[1-(3-amino-propyl)-piperidin-4-yl-methyl]-amino}-ethylester
19. Ölsäure-2-{(2-oleoyloxy-ethyl)-[1-(2-amino-ethyl)-piperidin-4-yl-methyl]-amino}-ethylester
20. 4-Dimethylamino-1-{3-{bis-(2-tetradecyloxy-ethyl)-aminol-propyl}-piperidin
21. 4'-{[Bis-(2-tetradecanoyloxy-ethyl)]-amino}-[4,1']bipiperidin-1-carboxamidin

### Vergleichsbeispiel 1

### Decansäure-2-{(2-decanoyloxy-ethyl)-[3-(4-methyl-piperazin-1-yl)-propyl]-amino}-ethylester-hydrochlorid.

Zu einer Lösung von 1.35 g (5.5 mmol) 2-{(2-Hydroxy-ethyl)-[3-(4-methylpiperazin-1-yl)-propyl]-amino}-ethanol in 30 ml Tetrahydrofuran gibt man 2.1 ml (15 mmol) Triethylamin, tropft die Lösung von 2.3 ml (11 mmol) Decansäurechlorid in 20 ml Tetrahydrofuran zu und erwärmt 20 h zum Rückfluß. Nach dem Abkühlen wird filtriert, das Filtrat eingeengt und der Rückstand an Kieselgel chromatographiert. Mit Ethylacetat/Methanol 1:1 eluiert man 1.28 g der gewünschten Verbindung als Öl, die in Ethylacetat gelöst und mit überschüssiger etherischer Chlorwasserstofflösung versetzt wird. Nach Einengen der Lösung filtriert man den Niederschlag ab und isoliert 1.3 g Titelverbindung (42% d.Th.) vom Schmp. 208-212°C.

Das als Ausgangsstoff verwendete 2-{(2-Hydroxy-ethyl)-[3-(4-methyl-piperazin-1-yl)-propyl]-amino}-ethanol kann wie folgt erhalten werden:
Zu einer Lösung aus 4.1 g (40 mmol) Diethanolamin und 7.8 g (44 mmol) 3-(4-Methyl-piperazin-1-yl)-propylchlorid in 40 ml Dimethylformamid gibt man 50 mg Kaliumiodid und erwärmt 5 h auf 60°C. Nach Einengen im Vakuum wird der Rückstand an Kieselgel chromatographiert. Mit Ethylacetat/Methanol 1:1 eluiert man 2.7 g (25% d.Th.) der gewünschten Verbindung als Öl.

### Vergleichsbeispiel 2

### Ölsäure-2-{(2-oleoyloxy-ethyl)-[3-(4-methyl-piperazin-1-yl)-propyl]-amino}-ethylester-hydrochlorid

In analoger Weise wie in Beispiel 1 beschrieben erhält man aus 2-{(2-Hydroxyethyl)-[3-(4-ntethyl-piperazin-1-yl)-propyl]-amino}-ethanol und Ölsäurechlorid die Titelverbindung mit 35% Ausbeute als Öl.

### Beispiel 1

### Ölsäure-2-[(2-oleoyloxy-ethyl)-(3,4,5,6-tetrahydro-2H-[1,4']bipyridinyl-4-yl)-amino]-ethylester-hydrochlorid

In analoger Weise wie in Beispiel 1 beschrieben erhält man aus 2-Hydroxyethyl-(3,4,5,6-tetrahydro-2*H* [1,4']bipyridinyl-4-yl)-amino]-ethanol und Ölsäurechlorid die Titelverbindung mit 53% Ausbeute als zähes Öl.

Das als Ausgangsstoff verwendete 2-Hydroxy-ethyl-(3,4,5,6-tetrahydro-2*H-*[1,4']bipyridinyl-4-yl)-amino]-ethanol kann wie folgt erhalten werden:
a) Eine Mischung aus 62.9 g (0.33 mol) 1-Benzyl-piperidin-4-on, 34.8 g Diethanolamin und 300 ml Toluol wird 2 h am Wasserabscheider zum Rückfluß erhitzt. Nach Abscheidung von 5 ml Wasser wird eingeengt und der Rückstand im Vakuum destilliert. Man isoliert 73.2 g 2-(8-Benzyl-1-oxa-4,8-diaza-spiro[4.5]dec-4-yl)-ethanol (80% d.Th.) vom Sdp._{0.08} 185-190°C.
b) Eine Lösung von 87.6 g (0.32 mol) der vorstehend beschriebenen Verbindung in 900 ml Methanol wird über 2 g Platindioxid und anschließend über 2 g 10-proz. Palladiumkohle bei 4 bar Wasserstoffdruck hydriert. Nach Aufnahme der berechneten Wasserstoffmenge wird filtriert und eingeengt. Es verbleiben 55.0 g (91% d.Th.) 2-[(2-Hydroxy-ethyl)-(piperidin-4-yt)-amino]-ethanol als Öl.
c) Eine Mischung aus 24 g (127 mmol) der vorstehend beschriebenen Verbindung und 7.2 g (64 mmol) 4.Chlor-pyridin wird 2 h auf 150°C erhitzt, anschließend in 10N Natronlauge aufgenommen und mit Dichlormethan und Methanol extrahiert. Nach
Einengen des Extrakts und Verreiben mit Ethylacetat verbleiben 14.3 g (91% d.Th.) 2-[2-Hydroxy-ethyl-(3,4,5,6-tetrahydro-2*H*-[1,4']bipyridinyl-4-yl)-amino]-ethanol vom Schmp. 126-128°C.

### Beispiel 2

### Tetradecansäure-2-[(2-tetradecanoyloxy-ethyl)-(3,4,5,6-tetrahydro-2H-[1,4']bipyridinyl-4-yl)-amino]-ethylester-hydrochlorid

In analoger Weise wie in Beispiel 1 beschrieben erhält man aus 2-[(2-Hydroxyethyl)-(3,4,5,6-tetrahydro-2*H*-[1,4']bipyridinyl-4-yl)-amino]-ethanol und Tetradecansäurechlorid die Titelverbindung mit 51% Ausbeute als amorphes Pulver vom Schmelzbereich 100-120°C.

### Beispiel 3

### Dodecansäure-2-[(2-dodecanoyloxy-ethyl)-(3,4,5,6-tetrahydro-2H-[1,4']bipyridinyl-4-yl)-amino]-ethylester-hydrochlorid

In analoger Weise wie in Beispiel 1 beschrieben erhält man aus 2-[(2-Hydroxyethyl)-(3,4,5,6-tetrahydro-2*H*-[1,4']bipyridinyl-4-yl)-amino]-ethanol und Dodecansäurechlorid die Titelverbindung mit 48% Ausbeute als amorphes Pulver .

### Beispiel 4

### Ölsäure-2-[(2-oleoyloxy-ethyl)-(3 ,4,5,6-tetrahydro-2H-[1,4']bipyridinyl-4-yl-methyl)-amino]-ethylester-hydrochlorid

In analoger Weise wie in Beispiel 1 beschrieben erhält man aus 2-[(2-Hydroxyethyl)-(3,4,5,6-tetrahydro-2*H*-[1,4']bipyridinyl-4-yl-methyl)-amino]-ethanol und Ölsäurechlorid die Titelverbindung mit 37% Ausbeute als Öl .

Das als Ausgangsstoff verwendete 2-Hydroxyethyl-(3,4,5,6-tetrahydro-2*H-*[1,4]bipyridinyl-4-yl)-amino]-ethanol kann wie folgt erhalten werden:
a) Eine Mischung aus 23.8 g (0.1 mol) 1 -Benzoyl-4-chlormethyl-piperidin und 21.0 g (0.1 mol) Diethanolamin wird 1 h auf 150°C erhitzt, anschließend mit 10N Natronlauge versetzt und mit Dichlormethan extrahiert. Nach dem Einengen verbleiben 30.4 g (99% d.Th.) 2-[(2-Hydroxy-ethyl)-(1-beazoyl-piperidin-4-yl-methyl)-amino]-ethanol als Öl.
b) 30.3 g der vorstehend beschriebenen Verbindung werden mit 200 ml 6N Salzsäure 5 h zum Rückfluß erhitzt. Nach dem Abkühlen wäscht man mit Diethylether, engt die wässrige Phase ein, stellt mit 10N Natronlauge alkalisch, extrahiert mit Dichlormethan, trocknet und engt ein. Es verbleiben 16.0 g (79% d.Th.) 2-[(2-Hydroxy-ethyl)-(piperidin-4-yl-methyl)-arnino]-ethanol als Öl.
c) Analog dem unter 3c) beschriebenen Verfahren erhält man aus der vorstehenden Verbindung und 4-Chlor-pyridin in 44% Ausbeute 2-[(2-Hydroxy-ethyl)-(3,4,5,6-tetrahydro-2*H*-[1,4']bipyridinyl-4-yl-methyl)-amino]-ethanol als Öl.

### Beispiel 5

### Tetradecansäure-2-[(2-tetradecanoyloxy-ethyl)-(3,4,5,6-tetmhydro-2H-[1,4']bipyridinyl-4-yl-methyl)-amino]-ethylester-hydrochlorid

In analoger Weise wie in Beispiel 1 beschrieben erhält man aus 2-[(2-Hydroxyethyl)-(3,4,5,6-tetrahydro-2*H*-[1,4']bipyridinyl-4-yl-methyl)-amino]-ethanol und Tetradecansäurechlorid die Titelverbindung mit 86% Ausbeute als Öl.

### Beispiel 6

### Dodecansäure-2-[(2-dodecanoyloxy-ethyl)-(3,4,5,6-tetrahydro-2H-[1,4']bipyridinyl-4-yl-methyl)-amino]-ethylester-hydrochlorid

In analoger Weise wie in Beispiel 1 beschrieben erhält man aus 2-[(2-Hydroxyethyl)-(3,4,5,6-tetrahydro-2*H*-[1,4']bipyridinyl-4-yl-methyl)-amino]-ethanol und Dodecansäurechlorid die Titelverbindung mit 81% Ausbeute als Öl.

### Vergleichsbeispiel 3

### Ölsäure-2-[(2-oleoyloxy-ethyl)-(3-piperidin-1-yl-propyl)-amino]-ethylester

In analoger Weise wie in Beispiel 1 beschrieben erhält man aus 2-[(2-Hydroxyethyl)-(3-piperidin-1-yl-propyl)-amino]-ethanol (J. Organomet. Chem. 251, 289 (1983)) und Ölsäurechlorid die Titelverbindung mit 39% Ausbeute als Öl.

### Beispiel 7

### Dodecansäure-2-{[3-(4-amino-piperidin-1-yl)-propyl]-(2-dodecanoyloxy-ethyl)-amino}-ethylester

Zu der Lösung von 0.7 g (2 mmol) 2-{[3-(4-Amino-piperidin-1-yl)-propyl]-(2-hydroxyethyl)-amino}-ethanol-hydrochlorid in 10 ml Trifluoressigsäure tropft man 1.0 g (5 mmol) Dodecansäurechlorid und rührt 18 h bei Raumtemperatur. Man engt ein, versetzt mit 10 ml kalter 1N Natronlauge, extrahiert mit Ethylacetat, engt ein und chromatographiert an Kieselgel. Mit Ethylacetat/Methanol 4:1 eluiert man 0.87 g (71% d.Th.) der gewünschten Verbindung als Öl.

Das als Ausgangsstoff verwendete 2-{[3-(4-Amino-piperidin-1-yl)-propyl]-(2-hydroxy-ethyl)-amino}-ethanol-hydrochlorid kann wie folgt erhalten werden:
a) Eine Mischung aus 56.2 g (0.2 mol) 4-Benzamido-1-(3-chlor-propyl)-piperidin. 19.8 g (0.19 mol) Diethanolamin, 16.6 g (0.12 mol) Kaliumcarbonat und 500 ml n-Propanol wird 32 h zum Rückfluß erhitzt. Man filtriert, engt das Filtrat ein und chromatographiert an Kieselgel. Mit Ethylacetät/Methanol 9:1 eluiert man 33.2 g (48% d.Th.) 2-{[3-(4-Benzamido-piperidin-1-yl)-propyl]-(2-hydroxy-ethyl)-amino}-ethanol als amorphen Feststoff.
b) 17.2 g (0.05 mol) der vorstehenden Verbindung werden mit 190 ml 6N Salzsäure 18 h zum Rückfluß erhitzt. Man läßt abkühlen, wäscht mit Dichlormethan und engt die wässrige Phase ein. Es verbleiben 17.0 g (96% d.Th.) 2-{[3-(4-Amino-piperidin-1-yl)-propyl]-(2-hydroxy-ethyl)-amino}-ethanol-hydrochlorid als Öl.

### Beispiel 8

### Tetradecansäure-2-{[3-(4-amino-piperidin-1-yl)-propyl]-(2-tetradecanoyloxy-ethyl)-amino} -ethylester

In analoger Weise wie in Beispiel 10 beschrieben erhält man aus 2- {[3-(4-Amino-piperidin-1-yl}-propyl]-(2-hydroxy-ethyl)-amino}-ethanol-hydrochlorid und Tetradecansäurechlorid die Titelverbindung mit 64% Ausbeute als Öl.

### Vergleichsbeispiel 4

### Ölsiure-2-(2-oleoyloxy-ethyl)-(2-piperidin-4-yl-ethyl)-amino]-ethylester-hydrochlorid

Eine Lösung von 0.95 g (3.8 mmol) 2-[(2-Hydroxy-ethyl)-(2-piperidin-4-yl-ethyl)-amino]-ethanol-hydrochlorid in 20 ml Dimethylformamid versetzt man mit 3.1 ml (9.5 mmol) Ölsäurechlorid und erwärmt 4 h auf 50°C. Man engt ein und chromatographiert an Kieselgel. Mit Ethylacetat/Methanol 9:1 eluiert man 0.4 g (14% d.Th.) der Titdverbindung als Öl.

Das als Ausgangsstoff verwendete 2-[(2-Hydroxy-ethyl)-(2-piperidin-4-yl-ethyl)-amino]-ethaiiol kann wie folgt erhalten werden:
Die Lösung von 57 g (0.27 mol) 2-[(2-Hydroxy-ethyl)-(2-pyridin-4-yl-ethyl)-amino]-ethanol (Chem. Abstr. 1960, 13129) in 700 ml Methanol wird mit 1.0 g aktiviertem Rutheniumoxid versetzt und bei 100°C und 150 bar Wasserstoffdruck 16 h hydriert. Man filtriert, engt ein und chromatographiert an Kieselgel. Mit Methanol eluiert man 47.9 g (82% d.Th.) der gewünschten Verbindung als Öl.

### Vergleichsbeispiel 5

### Tetradecansäure-2-[(2-tetradecanoyloxy-ethyl)-(2-piperidin-4-yl-ethyl)-amino]-ethylester

In analoger Weise wie in Beispiel 10 beschrieben erhält man aus 2-[(2-Hydroxyethyl)-(2-piperidin-4-yl-ethyl)-amino]-ethanol und Tetradecansäurechlorid die Titelverbindung mit 71% Ausbeute als Öl.

### Vergleichsbeispiel 6

### Dodecansäure-2-[(2-dodecanoyloxy-ethyl)-(2-piperidin-4-yl-ethyl)-amino]-ethylester

In analoger Weise wie in Beispiel 10 beschrieben erhält man aus 2-[(2-Hydroxyethyl)-(2-piperidin-4-yl-ethyl)amino]-ethanol und Dodecansäurechlorid die Titelverbindung mit 90% Ausbeute als Öl,

### Beispiel 9

### Tetradecansäure-2-{(2-tetradecanoyloxy-ethyl)-[2-(3,4,5,6-tetrahydro-2H-[1,4']bipyridinyl-4-yl)-ethyl]-amino}-ethylester

In analoger Weise wie in Beispiel 10 beschrieben erhält man aus 2-{(2-Hydroxyethyl)-[2-(3,4,5,6-tetrahydro-2*H*-[1,4']bipyridinyl-4-yl)-ethyl]-amino}-ethanol und Tetradecansäurechlorid die Titelverbindung mit 95% Ausbeute als Öl.

### Beispiel 10

### Dodecansäure-2-{(2-dodecanoyloxy-ethyl)-[2-(3,4,5,6-tetrahydro-2H-[1, 4']bipyridinyl-4-yl)-ethyl]-amino}-ethylester

In analoger Weise wie in Beispiel 10 beschrieben erhält man aus 2-{(2-Hydroxyethyl)-[2-(3,4,5,6-tetrahydro-2H-[1,4']bipyridinyl-4-yl)-ethyl]-amino}-ethanol und Dodecansäurechlorid die Titelverbindung mit 65% Ausbeute als Öl.

### Beispiel 11

### Tetradecansäure-2-[(2-tetradecanoyloxy-ethyl)-[1,4']bipiperidinyl-4-yl-amino]-ethylester

In analoger Weise wie in Beispiel 10 beschrieben erhält man aus 2-[(2-Hydroxyethyl)-[1,4']bipiperidinyl-4-yl-amino]-ethanol und Tetradecansäurechlorid die Titelverbindung mit 35% Ausbeute als Öl.

Das als Ausgangsstoff verwendete 2-[(2-Hydroxy-ethyl)-[1,4']bipiperidinyl-4-yl-amino]-ethanol kann wie folgt erhalten werden:

24 g (90 mmol) des unter 3c) beschriebenen 2-[2-Hydroxy-ethyl-(3,4,5,6-tetrahydro-2*H*-[1,4']bipyridinyl-4-yl)-amino]-ethanols werden analog dem unter Beispiel 12 beschriebenen Verfahren an Rutheniumoxid hydriert. Man isoliert 17 g (70% d.Th.) der gewünschten Verbindung als Öl.

### Beispiel 12

### Dodecansäure-2-[(2-dodecanoyloxy-ethyl)-[1,4']bipiperidinyl-4-yl-amino]-ethylester

In analoger Weise wie in Beispiel 10 beschrieben erhält man aus 2-[(2-Hydroxyethyl)-[1,4']bipiperidinyl-4-yl-amino]-ethanol und Dodecansäurechlorid die Titelverbindung mit 32% Ausbeute als Öl.

### Beispiel 13

### Ölsäure-2-{(2-oleoyloxy-ethyl)-[2-(3,4,5,6-tetrahydro-2H-[1,4']bipyridinyl-4-yl)-ethyl]-amino}-ethylester-hydrochlorid

In analoger Weise wie in Beispiel 1 beschrieben erhält man aus 2-{(2-Hydroxyethyl)-[2-(3,4,5,6-tetrahydro-2*H*-[1,4']bipyridinyl-4-yl)-ethyl]-amino]-ethanol und Ölsäurechlorid die Titelverbindung mit 57% Ausbeute als Öl.

### Beispiel 14

### Ölsaure-2-[(2-oleoyloxy-ethyl)-(1'-methyl-[1,4']bipiperidinyl-4-yl)-amino]-ethylester

In analoger Weise wie in Beispiel 1 beschrieben erhält man aus 2-[(2-Hydroxyethyl)-(1'-methyl-[1,4']bipipendinyl-4-yl)-amino]-ethanol und Ölsäurechlorid die Titelverbindung mit 37% Ausbeute als Öl.

Das als Ausgangsstoff verwendete 2-[(2-Hydroxy-ethyl)-(1'-methyl-[1,4']bipiperidinyl-4-yl)-amino]-ethanol kann wie folgt erhalten werden:
a) 5.4 g (20 mmol) des unter Beispiel 17 beschriebenen 2-[(2-Hydroxy-ethyl)-[1,4']bipiperidinyl-4-yl-amino]-ethanols werden mit 20 ml Ameisensäureethylester und 1 ml Wasser 5 h zum Rückfluß erhitzt und anschließend eingeengt. Es verbleiben 6.2 g (quantitativ) 2-[(2-Hydroxy-ethyl)-(1'-formyl-[1,4']bipiperidinyl-4-yl)-amino]-ethanol als Öl.
b) 6.0 g (20 mmol) der vorstehend beschriebenen Verbindung werden in eine Suspension von 2.9 g Lithiumtetrahydridoaluminat in 150 ml Tetrahydrofuran getropft und anschließend 3 h zum Rückfluß erhitzt. Nach Stehen über Nacht wird mit Ethylacetat und gesättigter Kochsalzlösung zersetzt, filtrien und das Filtrat eingeengt. Man chromatographiert an Kieselgel und eluiert mit Ethylacetatlmethanolischem Ammoniak 9: 1.8 g (32% d.Th.) 2-[(2-Hydroxy-ethyl)-(1'-methyl-[1,4']bipiperidinyl-4-yl)-amino]-ethanol als Öl.

### Beispiel 15

### Dodecansäure-2- {(2-dodecanoyloxy-ethyl)-[1-(3 -amino-propyl)-piperidin-4-yl]-amino}-ethylester

In analoger Weise wie in Beispiel 10 beschrieben erhält man aus 2-{(2-Hydroxyethyl)-[1-(3-amino-propyl)-piperidin-4-yl]-amino}-ethanol und Dodecansäurechlorid die Titelverbindung mit 37% Ausbeute als Öl.

Das als Ausgangsstoff verwendete 2-{(2-Hydroxy-ethyl)-[1-(3-amino-propyl)-piperidin-4-yl]-amino}-ethanol kann wie folgt erhalten werden:
a) Eine Mischung aus 5.5 g (29 mmol) des unter 3b) beschriebenen 2-[(2-Hydroxyethyl)-(piperidin-4-yl)-amino]-ethanols, 60 ml Methanol und 1.9 ml (29 mmol) Acrylnitril wird 24 h bei Raumtemperatur gerührt und anschließend im Vakuum eingeengt. Es verbleiben 6.7 g (96% d.Th.) 2-{(2-Hydroxy-ethyl)-[1-(2-cyanoethyl)-piperidin-4-yl]-amino}-ethanol als Öl.
b) 6.7 g (28 mmol) der vorstehend beschriebenen Verbindung werden in 150 ml methanolischem Ammoniak über Raney-Nickel bei 100°C und 100 bar Wasserstoffdruck hydriert. Man filtriert, engt ein und erhält 6.8 g (quantitativ) 2-{(2-Hydroxy-ethyl)-[1-(3-amino-propyl)-piperidin-4-yl]-amino}-ethanol als Öl.

### Beispiel 16

### Tetradecansäure-2-{(2-tetradecanoyloxy-ethyl)-[1-(3-amino-propyl)-piperidin-4-yl]-amino} -ethylester

In analoger Weise wie in Beispiel 10 beschrieben erhält man aus 2-{(2-Hydroxyethyl)-[1-(3-amino-propyl)-piperidin-4-yl]-amino}-ethanol und Tetradecansäurechlorid in 34% Ausbeute die Titelverbindung als Öl.

### Beispiel 17

### Dodecansäure-2-{(2-dodecanoytoxy-ethyl)-[1-(3-amino-propyl)-piperidin-4-yl-methyl]-amino}-ethylester

In analoger Weise wie in Beispiel 10 beschrieben erhält man aus 2-{(2-Hydroxyethyl)-[1-(3-amino-propyl)-piperidin-4-yl-methyl)-amino}-ethanol und Dodecansäurechlorid die Titelverbindung mit 44% Ausbeute als Öl.

Der verwendete Ausgangsstoff kann aus der Verbindung nach Beispiel 6b) durch Umsetzung mit Acrylnitril analog Beispiel 21a) und anschließende Hydrierung analog Beispiel 21b) erhalten werden.

### Beispiel 18

### Tetradecansäure-2-{(2-tetradecanoyloxy-ethyl)-[1-(3-amino-propyl)-piperidin-4-yl-methyl]-amino}-ethylester

In analoger Weise wie in Beispiel 10 beschrieben erhält man aus 2-{(2-Hydroxy-ethyl)-[1-(3-amino-propyl)-piperidin-4-yl-methyl]-amino}-ethanol und Tetradecansäurechlorid die Titelverbindung mit 38% Ausbeute als Öl.

### Beispiel 19

### Tetradecansäure-2-{(2-tetradecanoyloxy-ethyl)-[3-(3,4,5,6-tetrahydro-2H-[1,4']bipyridinyl-4-ylamino)-propyl]-amino}-ethylester

In analoger Weise wie in Beispiel 10 beschrieben erhält man aus 2-{(2-Hydroxyethyl)-[3-(3,4,5,6-tetrahydro-2*H*-[1,4']bipyridinyl-4-ylamino)-propyl]-amino}-ethanol und Tetradecansäurechlorid die Titelverbindung mit 29% Ausbeute als Öl.

Das als Ausgangsstoff verwendete 2-{(2-Hydroxy-ethyl)-[3-(3,4,5,6-tetrahydro-2*H*-[1,4']bipyridinyl-4-ylamino)-propyl]-amino}-ethanol kann wie folgt erhalten werden:
a) Eine Lösung von 46 g (0.4 mol) 4-Chlorpyridin und 123.5 g (0.86 mol) 4-Piperidon-ethylenketal wird in 400 ml p-Xylol 48 h zum Rückfluß erhitzt. Man kühlt ab, filtriert, engt das Filtrat ein und chromatographiert an Kieselgel. Mit Ethylacetat/ammoniakalisches Methanol 9:1 eluiert man 79.7 g (90% d.Th.) 8-Pyridin-4-yl-1,4-dioxa-8-aza-spiro[4.5]decan vom Schmp. 65°C.
b) Eine Lösung von 79.7 g des vorstehend beschriebenen Ketals in 21 Tetrahydrofuran wird mit 1000 ml 6N Salzsäure versetzt und 2 h bei Raumtemperatur gerührt. Man engt ein, stellt mit halbkonz. Ammoniakwasser basisch und extrahiert mit Dichlormethan. Nach Einengen des Extrakts verbleiben 64.2 g (quantitativ) 2,3,5,6-Tetrahydro[1,4']bipyridin-4-an vom Schmp. 102 °C.
c) Eine Mischung aus 15 g (85 mmol) des vorstehend beschriebenen Ketons, 13.8 g (85 mmol) 2-[3-Amino-propyl)-(2-hydroxy-ethyl)-amino]-ethanol (J. Am. Chem. Soc. 66, 728 (1944)), 100 mg 4-Toluolsulfonsäure und 200 ml Toluol wird 3 h am Wasserabscheider erhitzt und anschließend eingeengt. Man nimmt den Rückstand in 200 ml Methanol auf, fügt 500 mg Platindioxid zu und hydriert 10 h bei 1 bar Wasserstoffdruck. Nach Filtration und Einengen chromatographiert man an Kieselgel und eluiert mit Ethylacetat/Methanol 1:1 22.4 g 2-{(2-Hydroxy-ethyl)-[3-(3,4,5,6-tetrahydro-2*H*-[1,4']bipyridinyl-4-ylamino)-propyl]-amino}-ethanol als Öl.

### Beispiel 20

### Dodecansäure-2-{(2-dodecanoyloxy-ethyl)-[3-(3,4,5,6-tetrahydro-2H-[1,4']bipyridinyl-4-ylamino)-propyl]-anuno}-ethylester

In analoger Weise wie in Beispiel 10 beschrieben erhält man aus 2-{(2-Hydroxyethyl)-[3-(3,4,5,6-tetrahydro-2*H*-[1,4']bipyridinyl-4-ylamino)-propyl]-amino}-ethanol und Dodecansäurechlorid die Titelverbindung mit 34% Ausbeute als Öl.

### Beispiel 21

### Ölsäure-2-{(2-oleoyloxy-ethyl)-[1'-(3-dimethylamino-propyl)-[1,4']bipiperidinyl-4-yl]-amino }-ethylester

In analoger Weise wie in Beispiel 1 beschrieben erhält man aus 2-{(2-Hydroxyethyl)-[1'-(3-dimethylamino-propyl)-[1,4']bipiperidinyl-4-yl]-amino}-ethanol und Ölsäurechlorid die Titelverbindung mit 23% Ausbeute als Öl.

Das als Ausgangsstoff verwendete 2-{(2-Hydroxy-ethyl)-[1'-(3-dimethylaminopropyl)-[1,4']bipiperidinyl-4-yl]-amino}-ethanol kann wie folgt erhalten werden:
Eine Mischung aus 4.05 g (15 mmol) des unter Beispiel 17 beschriebenen 2-[(2-Hydroxy-ethyl)-[1,4']bipiperidinyl-4-yl-amino]-ethanols. 2.0 g Kaliumcarbonat, 2.2 g 3-Dimethylamino-propylchlorid und 25 ml n-Propanol wird 5 h zum Rückfluß erhitzt, abgekühlt, filtriert und das Filtrat eingeengt. Man chromatographiert an Kieselgel und eluiert mit Ethylacetat/methanolischem Ammoniak 1: 12.0 g (37% . d.Th.) der gewünschten Verbindung als Öl.

### Beispiel 22

### Ölsäure-2-{[3-(4-dimethylamino-pipendin-1-yl)-propyl]-(2-oleoyloxy-ethyl)-amino}-ethylester

In analoger Weise wie in Beispiel 1 beschrieben erhält man aus 2-{[3-(4-dimethylamino-piperidin-1-yl)-propyl]-(2-hydroxy-ethyl)-amino}-ethanol und Ölsäurechlorid die Titelverbindung mit 65% Ausbeute als Öl.

Das als Ausgangsstoff verwendete 2-{[3-(4-dimethylamino-piperidin-1-yl)-propyl]-(2-hydroxy-ethyl)-amino}-ethanol kann wie folgt erhalten werden:
a) Analog der unter Beispiel 12 beschriebenen Vorstufe erhält man aus 4-Dimethylamino-pyridin durch Hydrierung über Rutheniumoxid 4-Dimethylamino-piperidin mit 75% Ausbeute als Öl.
b) Eine Mischung aus 15.6 g (0.15 mol) Diethanolamin, 30.9 ml (0.3 mol) 1-Brom-3-chlor-propan, 300 ml Tetrahydrofuran und 12.6 g Kaliumcarbonat wird 5 h zum Rückfluß erwärmt, filtriert, eingeengt und an Kieselgel chromatographiert. Mit Ethylacetat/Methanol 9:1 eluiert man 16.7 g (62% d.Th.) N-(3-Chlor-propyl)-diethanolamin als Öl.
c) Eine Mischung aus 1.92 (15 mmol) des Piperidins a), 2.72 g (15 mmol) des Halogenids b), 1.2 g Kaliumcarbonat und 50 ml n-Propanol wird 6 h zum Rückfluß erhitzt, filtriert; eingeengt und an Kieselgel chromatographiert. Mit Ethylacetat/methanolischem Ammoniak 1:1 eluiert man 2.7 g (66% d.Th.) 2-{[3-(4-dimethylamino-piperidin-1-yl)-propyl]-(2-hydroxy-ethyl)-amino}-ethanol als Öl.

### Beispiel 23

### Ölsäure-2-{(2-oleoyloxy-ethy)-[1-(3-dimethylamino-propyl)-piperidin-4-yl]-amino} -ethylester

In analoger Weise wie in Beispiel 1 beschrieben erhält man aus 2-{(2-Hydroxyethyl)-[1-(3-dimethylamino-propyl)-piperidin-4-yl]-amino}-ethanol und Ölsäurechlorid die Titelverbindung mit 24% Ausbeute als Öl.

Das als Ausgangsstoff verwendete 2-{(2-Hydroxy-ethyl)-[1-(3-dimethylaminopropyl)-piperidin-4-yl]-amino}-ethanol kann wie folgt erhalten werden:
Eine Mischung aus 5.84 g (30 mmol) des unter 3b) beschriebenen 2-[(2-Hydroxyethyl)-(piperidin-4-yl)-amino]-ethanols, 4.0 g (33 mmol) 3-Dimethylamino-propylchlorid, 2.5 g Kaliumcarbonat und 20 ml n-Propanol wird 5 h zum Rückfluß erhitzt, filtriert, eingeengt und an Kieselgel chromatographiert. Mit Ethylacetat/methanolischem Ammoniak 1:1 eluiert man 4.4 g (54% d.Th.) der gewünschten Verbindung als Öl.

### Beispiel 24

### Ölsäure-2-{[3-(4-amino-piperidin-1-yl)-propyl]-(2-oleoyloxy-ethyl)-amino}-ethylester

Eine Suspension von 4.5 g (4 mmol) des unter Beispiel 10 beschriebenen 2-{[3-(4-Amino-piperidin-1-yl)-propylj-(2-hydroxy-ethyl)-amino}-ethanol-hydrochlorids in 100 ml Dichlormethan versetzt man mit 3.5 g N-Ethyl-diisopropylamin und 0.87 g (4 mmol) Pyrokohlensäuredi-t-butylester, erwärmt 18 h zum Rückfluß, tropft die Lösung von 2.4 g (8 mmol) Ölsäurechlorid in 40 ml Dichlormethan zu, erwärmt 18 h zum Rückfluß, versetzt mit 15 ml etherischer Chlorwasserstofflösung und rührt 6 h bei Raumtemperatur. Man engt ein, stellt mit N Natronlauge auf pH 9 ein, extrahiert mit Dichlormethan und Methanol, trocknet und engt ein. Nach Chromatographie an Kieselgel (Elutionsmittel Ethylacetat/Methanol 1 :1) isoliert man 1.1 g (36% d.Th.) der Titelverbindung als Öl.

### Beispiel 25

### Ölsäure-2-{[3-(4-aminomethyl-piperidin-1-yl)-propyl]-(2-oleoyloxy-ethyl)-amino}-ethylester

In analoger Weise wie in Beispiel 30 beschrieben erhält man aus 2-{[3-(4-Aminomethyl-piperidin-1-yl)-propyl]-(2-hydroxy-ethyl)-amino}-ethanol und Ölsäurechlorid die Titelverbindung mit 28% Ausbeute als Öl.

Das als Ausgangsstoff verwendete 2-{[3-(4-Aminomethyl-piperidin-1-yl)-propyl]-(2-hydroxy-ethyl)-amino}-ethanol kann analog zu der unter 28c) beschriebenen Verbindung aus 4-Aminomethyl-piperidin erhalten werden.

### Vergleichsbeispiel 7

### Ölsäure-2-[(2-oleoyloxy-ethyl)-(1'-ethyl-[1,4']bipiperidinyl-4-yl)-amino]-ethylester

In analoger Weise wie in Beispiel 1 beschrieben erhält man aus 2-[(2-Hydroxyethyl)-(1'-ethyl-[1,4']bipiperidinyl-4-yl)-amino]-ethanol und Ölsäurechlorid die Titelverbindung mit 22% Ausbeute als Öl.

Das als Ausgangsstoff verwendete 2-[(2-Hydroxy-ethyl)-(1'-ethyl-[1,4']bipiperidinyl-4-yl)-amino]-ethanol kann wie folgt erhalten werden:
a) 5.4 g (20 mmol) des unter Beispiel 17 beschriebenen 2-[(2-Hydroxy-ethyl)-[1,4']bipiperidinyl-4-yl-amino]-ethanols werden in 20 ml Dimethylformamid und 60 ml Dichlormethan mit 1.6 ml Acetylchlorid und 1.7 g Natriumhydrogencarbonat 5 h bei Raumtemperatur gerührt. Man filtriert, trocknet und engt ein. Es verbleiben 6.4 g (quantitativ) 2-[(2-Hydroxy-ethyl)-(1'-acetyl-[1,4']bipiperidinyl-4-yl)-amino]-ethanol als Öl.
b) 5.6 g (18 mmol) der vorstehend beschriebenen Verbindung werden analog Beispiel 20b) reduziert. Man erhält 2.1 g (39% d.Th.) 2-[(2-Hydroxy-ethyl)-(1'-ethyl-[1,4']bipiperidinyl-4-yl)-amino]-ethanol als Öl.

### Beispiel 26

### Ölsäure-2-{(2-oleoyloxy-ethyl)-[1-(3-amino-propyl)-piperidin-4-yl]-amino}-ethylester

In analoger Weise wie in Beispiel 30 beschrieben erhält man aus 2-{(2-Hydroxyethyl)-[1-(3-amino-propyl)-piperidin-1-yl]-amino}-ethanol (Bsp. 21b) und Ölsäurechlorid die Titelverbindung mit 25% Ausbeute als Öl.

### Beispiel 27

### Tetradecansäure-2-{(2-tetradecanoyloxy-ethyl)-[1-(3-dimethylamino-propyl)-piperidin-4-yl-methyl]-amino}-elhylester

In analoger Weise wie in Beispiel 10 beschrieben erhält man aus 2-{(2-Hydroxyethyl)-[1-(3-dimethylamino-propyl)-piperidin-4-yl-methyl]-amino}-ethanol und Tetradecansäurechlorid die Titelverbindung mit 31% Ausbeute als Öl.

Das als Ausgangsstoff verwendete 2-{(2-Hydroxy-ethyl)-[1-(3-dimethylaminopropyl)-piperidin-4-yl-methyl]-amino}-ethanol kann wie folgt erhalten werden:
Durch Alkylierung der unter Beispiel 6b) beschriebenen Verbindung mit 3-Dimethylamino-propylchlorid analog dem unter Beispiel 27 beschriebenen Verfahren erhält man mit 29% Ausbeute 2-{(2-Hydroxy-ethyl)-[1-(3-dimethylamino-propyl)-piperidin-4-yl-methyl]-amino}-ethanol als Öl.

### Beispiel 28

### Ölsäure-2- {(2-oleoyloxy-ethyl)-[1-(3-dimethylamino-propyl)-piperidin-4-yl-methyl]-amino}-ethylester

In analoger Weise wie in Beispiel 1 beschrieben erhält man aus 2-{(2-Hydroxyethyl)-[1-(3-dimethylamino-propyl)-piperidin-4-yl-methyl]-amino}-ethanol und Ölsäurechlorid die Titelverbindung mit 26% Ausbeute als Öl.

### Beispiel 29

### Tetradecansäure-2-{(2-tetradecanoyloxy-ethyl)-2-[[1-(3-amino-propyl)-piperidin-4-yl]-ethyl]-amino}-ethylester

In analoger Weise wie in Beispiel 10 beschrieben erhält man aus 2-{(2-Hydroxyethyl)-2-[[1-(3-amino-propyl)-piperidin-4-yl]-ethyl]-amino}-ethanol und Tetradecansäurechlorid die Titelverbindung mit 44% Ausbeute als Öl.

Das als Ausgangsstoff verwendete 2-{(2-Hydroxy-ethyl)-2-[[1-(3-amino-propyl)-piperidin-4-yl]-ethyl]-amino}-ethanol kann analog der unter Beispiel 21a) und 21b) beschriebenen Reaktionsfolge aus Acrylnitril und der Vorstufe des Beispiels 12 erhalten werden (Ausbeute 47%).

### Beispiel 30

### Dodecansäure-2-{(2-dodecanoyloxy-ethyl)-2-[[1-(3-amino-propyl)-piperidin-4-yl]-ethyl]-amino} -ethylester

In analoger Weise wie in Beispiel 10 beschrieben erhält man aus 2-{(2-Hydroxyethyl)-2-[[1-(3-amino-propyl)-piperidin-4-yl]-ethyl]-amino}-ethanol und Dodecansäurechlorid die Titelverbindung mit 61% Ausbeute als Öl.

### Beispiel 31

### Tetradecansäure-2-{(2-tetradecanoyloxy-ethyl)-2-[[1-(3-dimethylamino-propyl)-piperidin-4-yl]-ethyl]-amino}-ethylester

In analoger Weise wie in Beispiel 1 beschrieben erhält man aus 2-{(2-Hydroxyethyl)-2-[[1-(3-dimethylamino-propyl)-piperidin-4-yl]-ethyl]-amino}-ethanol und Tetradecansäurechlorid die Titelverbindung mit 50% Ausbeute als Öl.

Das als Ausgangsstoffverwendete 2-{(2-Hydroxy-ethyl)-2-[[1-(3-dimethylaminopropyl)-piperidin-4-yl]-ethyl]-amino}-ethanol kann aus der Vorstufe des Beispiels 12 und 3-Dimethylamino-propylchlorid analog der Vorstufe des Beispiels 27 mit 66% Ausbeute erhalten werden.

### Beispiel 32

### Ölsäure-2-{(2-oleoyloxy-ethyl)-2-[[1-(3-dimethylamino-propyl)-piperidin-4-yl]-ethyl]-amino}-ethylester

In analoger Weise wie in Beispiel 1 beschrieben erhält man aus 2-{(2-Hydroxyethyl)-2-[[1-(3-dimethylamino-propyl)-piperidin-4-yl]-ethyl]-amino)-ethanol und Olsäurechlorid die Titelverbindung mit 41% Ausbeute als Öl.

### Beispiel 33

### Tetradecansäure-2-{[3-(4-(2-amino-ethyl)-piperidin-1-yl)-propyl]-(2-tetradecanoyloxy-ethyl)amino}-ethylester

In analoger Weise wie in Beispiel 10 beschrieben erhält man aus 2-{[3-(4-(2-Amino-ethyl)-piperidin-1-yl)-propyl]-(2-hydroxy-ethyl)-amino}-ethanol und Tetradecansäurechlorid die Titelverbindung mit 32% d.Th. als Öl.

Das als Ausgangsstoff verwendete 2-{[3-(4-(2-Amino-ethyl)-piperidin-1-yl)-propyl]-(2-hydroxy-ethyl)-amino}-ethanol kann wie folgt erhalten werden:
a) Hydrierung von 4-(2-Amino-ethyl)-pyridin (J. Am. Chem. Soc. 78, 4129 (1956)) analog dem unter Beispiel 12 beschriebenen Verfahren ergibt mit 68% Ausbeute 4-(2-Anino-ethyl)-piperidin.
b) 9.0 g (70 mmol) der vorstehenden Verbindung werden mit 6.4 g (35 mmol) der Verbindung des Beispiels 28b) 1 h auf 150°C erhitzt. Man läßt abkühlen, nimmt in 15 ml 2N Natronlauge auf und stellt mit 10N Natronlauge stark alkalisch. Nach Extraktion mit Dichlormethan, Trocknen und Einengen des Extrakts wird an Kieselgel chromatographiert. Mit Ethylacetat/Methanot 3:1 eluiert man 6.0 g (63% d.Th.) 2-{[3-(4-(2-Amino-ethyl)-piperidin-1-yl)-propyl]-(2-hydroxy-ethyl)-amino}-ethanol.

### Beispiel 34

### Dodecansäure-2-{[3-(4-(2-amino-ethyl)-piperidin-1-yl)-propyl]-(2-dodecanoyloxy-ethyl)-amino}-ethylester

In analoger Weise wie in Beispiel 10 beschrieben erhält man aus 2-{[3-(4-(2-Amino-ethyl)-pipendin-1-yl)-propyl]-(2-hydroxy-ethyl)-amino}-ethanol und Dodecansäurechlorid die Titelverbindung mit 35% d.Th. als Öl.

### Beispiel 35

### Dodecansäure-2-{[3-(4-(3-amino-propylamino)-piperidin-(1-yl)-propyl]-(2-dodecanoyloxy-ethyl)-amino}-ethylester

In analoger Weise wie in Beispiel 10 beschrieben erhält man aus 2-{[3-(4-(3-Amino-propylamino)-piperidin-1-yl)-propyl]-(2-hydroxy-ethyl)-amino}-ethanol und Dodecansäurechlorid die Titelverbindung mit 18% d. Th. als Öl.

Das als Ausgangsstoff verwendete 2-{[3-(4-(3-Amino-propylamino)-piperidin-1-yl)-propyl]-(2-hydroxy-ethyl)-amino}-ethanol kann wie folgt erhalten werden:
Die Umsetzung des unter Beispiel 10 beschriebenen 2-{[3-(4-Amino-piperidin-1-yl)-propyl]-(2-hydroxy-ethyl)-amino}-ethanols mit Acrylnitril analog Beispiel 21a) und anschließende Hydrierung analog Beispiel 21b) ergibt die gewünschte Verbindung als Öl.

### Beispiel 36

### Tetradecansäure-2-{[3-(4-(3-amino-propylamino)-piperidin-1-yl)-propyl]-(2-tetradecanoyloxy-ethyl)-amino} -ethylester

In analoger Weise wie in Beispiel 10 beschrieben erhält man aus 2-{[3-(4-(3-Amino-propylamino)-piperidin-1-yl)-propyl]-(2-hydroxy-ethyl)-amino}-ethanol und Tetradecansäurechlorid die Titelverbindung mit 12% d.Th. als Öl.

### Beispiel 37

### 4-<2-{[Bis-(2-tetradecanoyloxy-ethyl)]-amino}-ethyl>-piperidin-1-carboxaraidin

Eine Mischung aus 1.92 g (3 mmol) Verbindung des Beispiels 13, 0.25 g Cyanamid und 5 ml n-Butanol wird 2 h auf 120°C erhitzt, abgekühlt, der Rückstand in Dichlormethan aufgenommen, mit wenig Wasser gewaschen, getrocknet und eingeengt. Nach Chromatographie an Kieselgel eluiert man mit Ethylacetat/Methanol 1:1 0.96 g (47% d.Th.) der Titelverbindung als Wachs.

### Beispiel 38

### N-[3-(4-<2-[Bis-(2-tetradecanoyloxy-ethyl)-amino]-ethyl>-piperidin-1-yl)-propyl]-guanidin

In analoger Weise wie in Beispiel 44 beschrieben erhält man aus der Verbindung des Beispiels 36 und Cyanamid die Titelverbindung mit 64% Ausbeute als Öl.

### Beispiel 39

### Ölsäure-2-[(2-ethylamino-ethyl)-(2-oleoyloxy-ethyl)-amino]-ethylester-hydrochlorid

In analoger Weise wie in Beispiel 1 beschrieben erhält man aus 2-[(2-Ethylamino-ethyl)-(2-hydroxy-ethyl)-amino]-ethanol und Ölsäurechlorid die Titelverbindung mit 36% Ausbeute als Öl.

Das als Ausgangsstoff verwendete 2-[(2-Ethylamino-ethyl)-(2-hydroxy-ethyl)-amino]-ethanol kann wie folgt erhalten werden:

11.0 g (58 mmol) N-{2-[Bis-(2-hydroxy-ethyl)-amino]-ethyl}-acetamid (J. Med. Chem. 36, 1839 (1993)) werden analog Beispiel 20b) reduziert. Man isoliert 8.3 g (81% d.Th.) der gewünschten Verbindung als Öl.

### Beispiel 40

### Ölsäure-2-[(2-diethylamino-ethyl)-(2-oleoyloxy-ethyl)-amino]-ethylester-hydrochlorid

In analoger Weise wie in Beispiel 12 beschrieben erhält man aus 2-[(2-Diethyl-amino-ethyl)-(2-hydroxy-ethyl)-aminol-ethanol und Ölsäurechlorid die Titelverbindung mit 44% Ausbeute als Öl.

### Beispiel 41

### Ölsäure-2-[(2-amino-ethyl)-(2-oteoyloxy-ethyl)-amino]-ethylester-hydrochlorid

In analoger Weise wie in Beispiel 12 beschrieben erhält man aus 2-[(2-Aminoethyl)-(2-hydroxy-ethyl)-amino]-ethanol (J. Am. Chem. Soc. 81, 3984 (1959)) und Ölsäurechlorid die Titelverbindung mit 25% Ausbeute als Öl.

### Beispiel 42

### Ölsäure-2-{[3-(3-amino-propylamino)-propyl]-(2-oleoyloxy-ethyl)-amino}-ethylester-hydrochlorid

In analoger Weise wie in Beispiel 12 beschrieben erhalt man aus 2-{[3-(3-Amino-propylamino)-propyl]-(2-hydroxy-ethyl)-amino}-ethanol und Ölsäurechlorid die Titelverbindung mit 29% Ausbeute als Öl.

Das als Ausgangsstoff verwendete 2-{[3-(3-Amino-propylamino)-propyl]-(2-hydroxy-ethyl)-amino}-ethanol kann durch Umsetzung von 2-[3-Amino-propyl)-(2-hydroxy-ethyl)-amino]-ethanol (J. Am. Chem. Soc. 66, 728 (1944)) mit Acrylnitril analog Beispiel 21a) und anschließende Hydrierung analog Beispiel 21b) erhalten werden. Sdp._{0.06} 176-177°C.

### Beispiel 43

### Ölsäure-2-[(3-dimethylamino-propyl)-(2-oleoyloxy-ethyl)-amino]-ethylesterhydrochlorid

In analoger Weise wie in Beispiel 1 beschrieben erhält man aus 2-[(3-Dimethylamino-propyl)-(2-hydroxy-ethyl)-amino]-ethanol und Olsäurechlorid die Titelverbindung mit 65% Ausbeute als Öl.

Das als Ausgangsstoff verwendete 2-[(3-Dimethylamino-propyl)-(2-hydroxyethyl)-amino]-ethanol kann analog dem unter Beispiel 27 beschriebenen Verfahren aus Diethanolamin und 3-Dimethylamino-propylchlorid erhalten werden. Sdp._{1.5} 135-136°C.

### Beispiel 44

### Ölsäure-2-[(3-diethylamino-propyl)-(2-olcoyloxy-ethyl)-amino]-ethylester

In analoger Weise wie in Beispiel 1 beschrieben erhält man aus 2-[(3-Diethylamino-propyl)-(2-hydroxy-ethyl)-amino]-ethanol (Chem. Pharm. Bull. 9, 313 (1961)) und Ölsäurechlorid die Titelverbindung mit 65% Ausbeute als Öl.

### Beispiel 45

### Tetradecansäure-2-[(3-dimethylamino-propyl)-(2-tetradecanoyloxy-ethyl)-amino]-ethylester

In analoger Weise wie in Beispiel 1 beschrieben erhält man aus 2-[(3-Dimethylamino-propyl)-(2-hydroxy-ethyl)-amino]-ethanol und Tetradecansäurechlorid die Titelverbindung mit 78% Ausbeute als Öl.

### Beispiel 46

### Dodecansäure-2-[(3-dimethylamino-propyl)-(2-dodecanoyloxy-ethyl)-amino]-ethylester

In analoger Weise wie in Beispiel 1 beschrieben erhält man aus 2-[(3-Dimethylamino-propyl)-(2-hydroxy-ethyl)-amino]-ethanol und Dodecansäurechlorid die Titelverbindung mit 79% Ausbeute als Öl.

### Beispiel 47

### Tetradecansäure-2-[(3-diethylamino-propyl)-(2-tetradecanoyloxy-ethyl)-amino]-ethylester

In analoger Weise wie in Beispiel 1 beschrieben erhält man aus 2-[(3-Diethylamino-propyl)-(2-hydroxy-ethyl)-amino]-ethanol und Tetradecansäurechlorid die Titelverbindung mit 50% Ausbeute als Öl.

### Beispiel 48

### Dodecansäure-2-[(3-diethylamino-propyl)-(2-dodecanoyloxy-ethyl)-amino]-ethylester

In analoger Weise wie in Beispiel 1 beschrieben erhält man aus 2-[(3-Diethylamino-propyl)-(2-hydroxy-ethyl)-amino]-ethanol und Dodecansäurechlorid die Titelverbindung mit 60% Ausbeute als Öl.

### Beispiel 49

### Ötsäure-2-[{3-[(3-dimethytamino-propyl)-methyl-amino]-propyl}-(2-oteoyloxy-ethyl)-amino]-ethylester

In analoger Weise wie in Beispiel 1 beschrieben erhält man aus 2-[{3-[(3-Dimethylamino-propyl)-methyl-amino]-propyl}-(2-hydroxy-ethyl)-amino]-ethanol und Ölsäurechlorid die Titelverbindung mit 63% Ausbeute als Öl.

Das als Ausgangsstoff verwendete 2-[{3-[(3-Dimethylamino-propyl)-methyl-amino]-propyl}-(2-hydroxy-ethyl)-amino]-ethanol kann durch Alkylierung von N,N,N'-Trimethyl-propan-1,3-diamin (J. Chem. Soc. (C) 1966, 527) mit N-(3-Chlor-propyl)-diethanolamin analog Beispiel 28c) erhalten werden.

### Beispiel 50

### Olsäure-2-[{3-[(3-diethylamino-propyl)-methyl-amino]-propyl}-(2-oleoyloxy-ethyl)-amino]-ethylester

In analoger Weise wie in Beispiel 1 beschrieben erhält man aus 2-[{3-[(3-Diethylamino-propyl)-methyl-amino]-propyl}-(2-hydroxy-ethyl)-amino]-ethanol und Ölsäurechlorid die Titelverbindung mit 59% Ausbeute als Öl.

Das als Ausgangsstoff verwendete 2-[{3-[(3-Diethylamnino-propyl)-methyl-amino]-propyl}-(2-hydroxy-ethyl)-amino]-ethanol kann analog der unter Beispiel 56 beschriebenen Vorstufe durch Alkylierung von N,N-Diethyl-N'-methyl-propan-1,3-diamin (Monatsh. Chem. 112, 825 (1981)) mit N-(3-Chlor-propyl)-diethanolamin erhalten werden.

### Beispiel 51

### Dodecansäure-2-{[4-(3-amino-propylamino)-butyl]-(2-dodecanoyloxy-ethyl)-amino}-ethylester-hydrochlorid

In analoger Weise wie in Beispiel 10 beschrieben erhält man die Titelverbindung aus 2-{[4-(3-Amino-propylamino)-butyl]-(2-hydroxy-ethyl)-amino}-ethanol und Dodecansäurechlorid mit 3 1 % Ausbeute als Wachs.

Das als Ausgangsstoff verwendete 2-{[4-(3-Amino-propylamino)-butyl]-(2-hydroxy-ethyl)-amino}-ethanol kann aus 2-[(4-Amino-butyl)-(2-hydroxy-ethyl)-amino]-ethanol (J. Am. Chem. Soc. 81, 3984 (1959)) und Acrylnitril analog Beispiel 21a) und anschließender Hydrierung analog Beispiel 21b) erhalten werden.

### Beispiel 52

### Dodecansäure-2-{[3-(3-amino-propylamino)-propyl]-(2-dodecanoyloxy-ethyl)-amino}-ethylester-hydrochlorid

In analoger Weise wie in Beispiel 10 beschrieben erhält man die Titelverbindung aus 2-{[3-(3-Amino-propylamino)-propyl]-(2-hydroxy-ethyl)-amino}-ethanol (siehe Beispiel 49) und Dodecansäurechlorid mit 49% Ausbeute vom Schmp. 245-246°C.

### Beispiel 53

### Tetradecansäure-2-{[4-(3-amino-propylamino)-butyl]-(2-tetradecanoyloxy-ethyl)-amino}-ethylester

In analoger Weise wie in Beispiel 10 beschrieben erhält man die Titelverbindung aus 2-{[4-(3-Amino-propylamino)-butyl]-(2-hydroxy-ethyl)-amino}-ethanol und Tetradecansäurechlorid mit 27% Ausbeute als Öl.

### Beispiel 54

### Tetradecansäure-2-[{3-[(3-dimethylamino-propyl)-methyl-amino]-propyl}-(2-tetradecanoyloxy-ethyl)-amino]-ethylester

In analoger Weise wie in Beispiel 1 beschrieben erhält man aus 2-[{3-[(3-Dimethylamino-propyl)-methyl-amino]-propyl}-(2-hydroxy-ethyl)-amino]-ethanol und Tetradecansäurechlorid die Titelverbindung mit 35% Ausbeute als Öl.

### Beispiel 55

### Tetradecansäure-2-[{3-[(3-diethylamino-propyl)-methyl-amino]-propyl}-(2-tetradecanoyloxy-ethyl)-amino]-ethylester

In analoger Weise wie in Beispiel 1 beschrieben erhält man aus 2-[{3-[(3-Diethylamino-propyl)-methyl-amino]-propyl}-(2-hydroxy-ethyl)-amino]-ethanol und Tetradecansäurechlorid die Titelverbindung mit 61% Ausbeute als Öl.

### Beispiel 56

### N-<3-{4-[Bis-(2-tetradecanoyloxy-ethyl)-amino]-piperidin-1-yl}-propyl>-guanidin

In analoger Weise wie in Beispiel 44 beschrieben erhält man aus der Verbindung des Beispiels 22 und Cyanamid die Titelverbindung mit 91% Ausbeute als Öl.

### Beispiel 57

### N-[3-(4-<[Bis-(2-tetradecanoyloxy-ethyl)-amino]-methyl>-piperidin-1-yl)-propyl]-guanidin

In analoger Weise wie in Beispiel 44 beschrieben erhält man aus der Verbindung des Beispiels 24 und Cyanamid die Titelverbindung mit 82% Ausbeute als Öl.

### Beispiel 58

### N-[2-(1-<3-[Bis-(2-tetradecanoyloxy-ethyl)-amino]-propyl>-piperidin-4-yl)-ethyl]-guanidin

In analoger Weise wie in Beispiel 44 beschrieben erhält man aus der Verbindung des Beispiels 40 und Cyanamid die Titelverbindung mit 94% Ausbeute als Öl.

### Beispiel 59

### Tetradecansäure-2- {(2-tetradecanoyloxy-ethyl)-[1-(2-amino-ethyl)-piperidin-4-yl]-amino} -ethylester

In analoger Weise wie in Beispiel 10 beschrieben erhält man aus 2-{(2-Hydroxyethyl)-[1-(2-amino-ethyl)-piperidin-4-yl]-amino}-ethanol und Tetradecansäurechlorid die Titelverbindung mit 38% Ausbeute als Öl.

Das als Ausgangsstoff verwendete 2-{(2-Hydroxy-ethyl)-[1-(2-amino-ethyl)-piperidin-4-yl]-amino}-ethanol kann wie folgt erhalten werden:
a) Eine Mischung aus 8.5 g (45 mmol) des unter Beispiel 3b) beschriebenen 2-[(2-Hydroxy-ethyl)-(piperidin-4-yl)-amino]-ethanols, 150 ml Dimethylformamid, 3.4 g (45 mmol) Chloracetonitril, 14.5g Kaliumcarbonat und 100 mg Kaliumiodid wird 2 h bei 60°C gerührt. Man filtriert und engt das Filtrat im Vakuum ein. Es verbleiben 10.5 g (quantitativ) 2-{(2-Hydroxy-ethyl)-[1-cyanomethyl-pipendin-4-yl]-amino}-ethanol als öliges Rohprodukt.
b) 4.1 g (18 mmol) der vorstehend beschriebenen Verbindung werden in 175 ml methanolischem Ammoniak über Raney-Nickel bei 35°C und 100 bar Wasserstoffdruck hydriert. Man filtriert, engt ein und erhält nach Chromatographie an Kieselgel 2.3 g (55% d.Th.) 2-{(2-Hydroxy-ethyl)-[1-(2-amino-ethyl)-piperidin-4-yl]-amino}-ethanol als Öl.

### Beispiel 60

### Tetradecansäure-2-{(2-tetradecanoyloxy-ethyl)-[1-(2-amino-ethyl)-piperidin-4-yl-methyl]-amino}-ethylester

In analoger Weise wie in Beispiel 10 beschrieben erhält man aus 2-{(2-Hydroxyethyl)-[1-(2-amino-ethyl)-piperidin-4-yl-methyl]-amino}-ethanol und Tetradecansäurechlorid die Titelverbindung mit 29% Ausbeute als Öl.

Das als Ausgangsstoff verwendete 2-{(2-Hydroxy-ethyl)-[1-(2-amino-ethyl)-piperidin-4-yl-methyl]-amino}-ethanol kann wie folgt erhalten werden:
a) Das unter Beispiel 6b) beschriebene Zwischenprodukt wird analog der unter Beispiel 66a) beschriebenen Methode mit Chloracetonitril umgesetzt.
b) Das vorstehend erhaltene Rohprodukt wird wie unter Beispiel 66b) hydriert. Man erhält das gewünschte 2-{(2-Hydroxy-ethyl)-[1-(2-amino-ethyl)-piperidin-4-yl-methyl]-amino}-ethanol mit 47% d.Th..

### Beispiel 61

### Tetradecansäure-2-{[3 -(4-(aminomethyl)-piperidin-1-yl)-propyl]-(2-tetradecanoyloxy-ethyl)-amino}-ethylester

In analoger Weise wie in Beispiel 10 beschrieben erhält man aus 2-{[3-(4-(Aminomethyl)-piperidin-1-yl)-propyl]-(2-hydroxy-ethyl)-amino}-ethanol und Tetradecansäurechlorid die Titelverbindung mit 27% Ausbeute als Öl.

Das als Ausgangsstoff verwendete 2-{[3-(4-Aminomethyl-piperidin-1-yl)-propyl]-(2-hydroxy-ethyl)-amino} -ethanol kann analog zu der unter Beispiel 28c) beschriebenen Verbindung aus 4-Aminomethyl-piperidin erhalten werden.

### Beispiel 62

### Tetradecansäure-2-{(2-tetradecanoyloxy-ethyl)-[1-(3-dimethylamino-propyl)-piperidin-4-yl]-amino} -ethylester

In analoger Weise wie in Beispiel 10 beschrieben erhält man aus 2-{(2-Hydroxyethyl)-[1-(3-dimethylamino-propyl)-piperidin-4-yl]-amino}-ethanol und Tetradecansäurechlorid die Titelverbindung mit 25% Ausbeute als Öl.

Das als Ausgangsstoff verwendete 2-{(2-Hydroxy-ethyl)-[1-(3-dimethylaminopropyl)-piperidin-4-yl]-amino}-ethanol ist unter Beispiel 29 beschrieben.

### Beispiel 63

### Teradecansäure-2-{[3-(4-dimethylamino-pipendin-1-yl)-propyl]-(2-tetradecanoyloxy-ethyl)-amino} -ethylester

In analoger Weise wie in Beispiel 10 beschrieben erhält man aus 2-{[3-(4-dimethylamino-piperidin-1-yl)-propyl]-(2-hydroxy-ethyl)-amino}-ethanol und Tetradecansäurechlorid die Titelverbindung mit 62% Ausbeute als Öl.

Das als Ausgangsstoff verwendete 2-{[3-(a-dimethylamino-pipezidin-1-yl)-propyl]-(2-hydroxy-ethyl)-amino}-ethanol ist unter Beispiel 28 beschrieben.

### Beispiel 64

### Tetradecansäure-2-{(2-tetradecanoymoxy-ethy)-[1-(4-amino-butyl)-piperidin-4-yl]-amino}-ethylester

In analoger Weise wie in Beispiel 10 beschrieben erhält man aus 2-{(2-Hydroxyethyl)-[1-(4-amino-butyl)-piperidin-4-yl]-amino}-ethanol und Tetradecansäurechlorid in 22% Ausbeute die Titelverbindung als Öl.

Das als Ausgangsstoff verwendete 2-{(2-Hydroxy-ethyl)-[1-(4-amino-butyl)-piperidin-4-yl]-amino}-ethanol kann wie folgt erhalten werden:
a) Eine Mischung aus 18.8 g (100 mmol) des unter Beispiel 3b) beschriebenen 2-[(2-Hydroxy-ethyl)-(piperidin-4-yl)-amino]-ethanols, 100 ml Dimethylformamid, 11 ml (110 mmol) 4-Brom-butyronitril, 18.9 ml N-Ethyldiisopropylamin und 100 mg 4-Dimethylamino-pyridin wird 8 h bei 90 °C gerührt und anschließend im Vakuum eingeengt. Man nimmt in Dichlormethan auf, filtriert und engt das Filtrat ein. Es verbleiben 15.0 g (59% d.Th.) 2-{(2-Hydroxy-ethyl)-[1-(3-cyano-propyl)-piperidin-4-yl]-amino}-ethanol als Öl.
b) 15.0 g (59 mmol) der vorstehend beschriebenen Verbindung werden in 300 ml methanolischem Ammoniak über Raney-Nckel bei 35 °C und 100 bar Wasserstoffdruck hydriert. Man filtriert, engt ein und erhält 10.9 g (71% d.Th.) 2-{(2-Hydroxy-ethyl)-[1-(4-amino-butyl)-piperidin-4-yl]-amino}-ethanol als Öl.

### Beispiel 65

### Tetradecansäure-2-{(2-tetradecanoyloxy-ethyl)-[1-(4-dimethylamino-butyl)-piperidin-4-yl]-amino}-ethylester

In analoger Weise wie in Beispiel 10 beschrieben erhält man aus 2-{(2-Hydroxyethyl)-[1-(4-dimethylamino-butyl)-piperidin-4-yl]-amino}-ethanol und Tetradecansäurechlorid in 23% Ausbeute die Titelverbindung als Öl.

Das als Ausgangsstoff verwendete 2-{(2-Hydroxy-ethyl)-[1-(4-dimethylamino-butyl)-piperidin-4-yl]-amino}-ethanol kann wie folgt erhalten werden:
Zu 6.5 g (25 mmol) 2-{(2-Hydroxy-ethyl)-[1-(4-amino-butyl)-piperidin-4-yl]-amino}-ethanol (Beispiel 71b) tropft man bei 0 °C 4.7 ml Ameisensäure und 5.6 ml gesättigte Formalinlösung, erwärmt auf 95 - 100 °C und rührt 9 h nach. Man läßt abkühlen, versetzt mit 6.5 ml konz. Salzsäure, erhitzt 3 h zum Rückfluß, stellt mit 10N Natronlauge stark alkalisch und extrahiert mit Dichlormethan. Nach Trocknen und Einengen des Extrakts verbleiben 6.0 g (84% d.Th.) der gewünschten Verbindung als Öl.

### Beispiel 66

### Tetradecansäure-2-{(2-tetradecanoyloxy-ethyl)-[1-(4-amino-butyl)-piperidin-4-yl-methyl]-amino} -ethylester

In analoger Weise wie in Beispiel 10 beschrieben erhält man aus 2-{(2-Hydroxyethyl)-[1-(4-amino-butyl)-piperidin-4-yl-methyl]-amino}-ethanol und Tetradecansäurechlorid in 33% Ausbeute die Titelverbindung als Öl.

Das als Ausgangsstoff verwendete 2-{(2-Hydroxy-ethyl)-[1-(4-amino-butyl)-piperidin-4-yl-methyl]-amino}-ethanol kann wie folgt erhalten werden:
Umsetzung von 2-[(2-Hydroxy-ethyl)-(piperidin-4-yl-methyl)-amino]-ethanol (Beispiel 6b) mit 4-Brom-butyronitril Analog Beispiel 71a) und anschließende Hydrierung analog Beispiel 71b) ergibt die gewünschte Verbindung als Öl.

### Beispiel 67

### Tetradecansäure-2- {(2-tetradecanoyloxy-ethyl)-[1-(4-dimethylamino-butyl)-piperidin-4-yl-methyl]-amino}-ethylester

In analoger Weise wie in Beispiel 10 beschrieben erhält man aus 2-{(2-Hydroxyethyl)-[1-(4-dimethylamino-butyl)-piperidin-4-yl-methyl]-amino}-ethanol und Tetradecansäurechlorid in 43% Ausbeute die Titelverbindung als Öl.

Das als Ausgangsstoff verwendete 2-{(2-Hydroxy-ethyl)-[1-(4-dimethylamino-butyl)-piperidin-4-yl-methyl]-amino}-ethanol kann aus dem unter Beispiel 73 beschriebenen 2-{(2-Hydroxy-ethyl)-[1-(4-amino-butyl)-piperidin-4-yl-methyl]-amino}-ethanol nach der unter Beispiel 72 beschriebenen Methode durch Umsetzung mit Ameisensäure und Formalinlösung erhalten werden (Ausbeute 69% d.Th.).

### Beispiel 68

### Tetradecansäure-2- {(2-tetradecanoyloxy-ethyl)-2-[[1-(4-amino-butyl)-piperidin-4-yl]-ethyl]-amino}-ethylester

In analoger Weise wie in Beispiel 10 beschrieben erhält man aus 2-{(2-Hydroxyethyl)- 2-[[1-(4-amino-butyl)-piperidin-4-yl]-ethyl]-amino}-ethanol und Tetradecansäurechlorid in 17% Ausbeute die Titelverbindung als Öl.

Das als Ausgangsstoff verwendete 2-{(2-Hydroxy-ethyl)- 2-[[1-(4-amino-butyl)-piperidin-4-yl]-ethyl]-amino}-ethanol kann wie folgt erhalten werden:
Die Umsetzung des unter Beispiel 12 beschriebenen 2-[(2-Hydroxy-ethyl)-(2-piperidin-4-yl-ethyl)-amino]-ethanol mit 4-Brom-butyronitril analog Beispiel 71a) und anschließende Hydrierung analog Beispiel 71b) ergibt die gewünschte Verbindung als Öl.

### Beispiel 69

### Tetradecansäure-2-{(2-tetradecanoyloxy-ethyl)-2-[[1-(4-dimethylamino-butyl)-piperidin-4-yl]-ethyl]-amino}-ethylester

In analoger Weise wie in Beispiel 10 beschrieben erhält man aus 2-{(2-Hydroxyethyl)- 2-[[1-(4-dimethylamino-butyl)-piperidin-4-yl]-ethyl]-amin}-ethanol und Tetradecansäurechlorid in 33% Ausbeute die Titelverbindung als Öl.

Das als Ausgangsstoffverwendete 2-{(2-Hydroxy-ethyl)-2-[[1-(4-dimethylamino-butyl)-piperidin-4-yl]-ethyl]-amino}-ethanol kann aus dem unter Beispiel 75 beschriebenen 2-{(2-Hydroxy-ethyl)-2-[[1-(4-amino-butyl)-piperidin-4-yl]-ethyl]-amino}-ethanol nach der unter Beispiel 72 beschriebenen Methode durch Umsetzung mit Ameisensäure und Formalinlösung erhalten werden (Ausbeute 72% d.Th.).

### Beispiel 70 Pharmakologische Testung

### 1.Testprinzip

Die Testung von DOTAP bzw. den Verbindungen der Erfindung besteht aus der Transfektion der Testzellen, der Proteinbestimmung mit der BCA-Methode (Pierce) und der Durchführung des CAT-Elisas und sind hier beispielhaft mit DOTAP (Boehringer Mannheim) beschrieben. Das kationische Lipid DOTAP bildet in wässriger Lösung nach Ultrabeschallung unilamellare Vesikel (Liposomen) aus, die sich mit der DNA (pCMV-CAT) spontan zu stabilen Komplexen formieren. Diese Komplexe adherieren an die Zelloberfläche, fusionieren mit der Zellmembran und pCMV-CAT wird ins Zytoplasma freigesetzt. Das transient exprimierte CAT wird im Zellysat nachgewiesen.

### 1.1. Testzellen

HeLa, humane Krebsepithelzellinie aus dem Gebärmutterhals, ATCC CCL 2 RPMI 1640, humane Krebsepithelzellinie aus der Nasenscheidewand, ATCC CCL 30
CALU 1, humane Krebsepithelzellinie aus der Lunge, ECACC 93120818 bzw. eine andere geeignete Testzellinie

### 1.2 Testmedium

Das für die jeweilige Zellinie verwendete Testmedium entspricht in der Zusammensetzung dem jeweiligen Kulturmedium, es wird nur der Gehalt an FKS um 50% verringert (anstatt 10% nur 5% FKS-Gehalt).

### 2. Durchführung der Bestimmung

### 2.1 Zelleinsaat für den Test

- in jedes well einer 6-well Multischale ewrden 2,0ml Zellsuspension (1,5x10⁵ Zellen/ml) gegeben
- 24h bei 37°C, 5% CO₂ inkubieren

### 2.2 Transfektionsansatz

Die DOTAP/DNA Komplexe werden in steriler 96-well Rundbodenplatte gemischt.
Beispiel:
Es werden 60µl HBS (Hank's buffered saline)-Puffer vorgelegt, dann 20µl DOTAP zugegeben. Die DNA wird mirt HBS 1:20 verdünnt. Dann werden 40µl =2µg DNA zu den Transfektionsansätzen gegeben und grundlich vermischt. Das Gemisch wird 15min bei Raumtemperatur stehen gelassen.

Durchführung der Transfektion:
- TM (Transfektionsmedium) aus allen wells absaugen
- 2ml TM/well zugeben
- rasche Zugabe der Transfektionsansätze
- einmal den Inhalt der wells durch leichtes Schwenken gut durchmischen
- Inkubation der Zellen 6h bei 37°C, 5%CO₂
- danach DOTAP/DNA-Gemisch absaugen
- Zugabe von 2ml TM/well
- Inkubation der Zellen 40-44h, 37°C, 5%CO₂

### 2.3 Lyse der Zellen

- TM absaugen und Zellen 2x mit 2ml eiskaltem PBS/well waschen, vollständig absaugen
- 0,5ml Lysispuffer (aus dem CAT-Elisa Kit, Fa. Boehringer Mannheim) zu den gewaschenen Zellen geben und 30min beiRT stehen lassen
- nach 30min die Lysate in Eppendorfcups uberführen und 10min bei 13000U mit Biofuge zentrifugieren
- aus dem Überstand wird ein Aliquot für den Proteinassay mit der BCA-Methode entnommen. Der Rest wird mit flüssigem Stickstoff schockgefroren und bis zur Durchführung des CAT-Elisas bei -80°C gelagert.

### 2.4 Proteinbestimmung der Lysate mit der BCA (Bicinochronic acid)-Methode

- mit der im Kit (Fa. Boehringer Mannheim) enthaltenen BSA (Rinderserumalbumin)-Lösung werden Proteinstandards durch Verdünnung mit Lysepuffer hergestellt
- 10µl der Proteinstandardlösung, des Leerweerts (Lysepuffer) und der unbekannten Probe werden in eine Mikrotiterplatte einpipettiert. Zu jedem well werden 200µl der Arbeitslösung zugefügt und die Platte 30min auf dem Schüttler geschüttelt.Nach 30min Inkubation bei 37°C wird bei550nm mit dem ELISA-Reader gemessen. Die Bestimmung der Proteinkonzentration erfolgt mit einem Auswerteprogramm.

### 2.5 CAT-Elisa

### Prinzip:

Der CAT (Chloramphenicolacetyltransferase)-Elisa dient der quantitativen Bestimmung der CAT-Expression in eukaryontischen Zellen nach Transfektion mit einem Plasmid, das CAT als Reportergen enthält. Der CAT-Elisa ist ein Sandwich-Enzym-Immunoassay.Anti-CAT-Antikörper sind adsorptiv an die Wände der Module gebunden. Im ersten Schritt bindet CATaus Zellextrakten spezifisch an die beschichteten Module. Im zweiten Schritt wird das fixierte CAT durch einen ANTI-CAT Antikörper, der mit Digoxigenin markiert ist (Anti-CAT-DIG), gebunden.Anti-CAT-DIG wird im dritten Schritt mit einem Peroxidase markiertem Antikörper gegen Digoxigenin (Anti.DIG-POD) detektiert und in einer nachfolgenden Substratreaktion visualisiert.

### Durchführung:

Die Arbeitsschritte des CAT-Elisas erfolgen gemäß der im Kit enthaltenen Arbeitsvorschrift.
Die Messung der Mikrotiterplatte erfolgt bei 405nm und einer Referenzwellenlänge von 492nm mit einem Elisa-Reader.

Die pharmakologischen Daten sind in folgender Tabelle beispielhaft gezeigt:

| relative Transfektionseffizienz in CAT-Assay (DOTAP = 1) | | |
|---|---|---|
| Verbindung von Beispiel | HeLa-Zellen | Calu-Zellen |
| 34 | 3.2 | 3.9 |
| 60 | 3.2 | 3.4 |

## Patentansprüche

1. Verbindungen der Formel 1 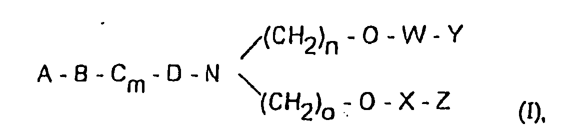 zur Verwendung als Arzneimittel,
in
A eine Gruppe NR₁R₂, eine Gruppe NR₁(CH₂)ₚNR₃R₄, eine Gruppe (C=NH)NH₂ oder einen Pyridinylrest.
B und D gleich oder verschieden jeweils eine Bindung, einen C₁- bis C₆-Alkylenrest oder eine Gruppe NR₅-C₂- bis C₆-Alkylen.
C Piperidindiyl oder Piperazindiyl.
W und X gleich oder verschieden jeweils eine Bindung oder eine Carbonylgruppe.
Y und Z gleich oder verschieden jeweils einen gesättigren oder ungesättigten Kohlenwasserstoffrest mit 7 bis 24 Kohlenstoffatomen.
R₁ bis R₁ gleich oder verschieden jeweils Wasserstoff oder einen C₁- bis C₆-Alkylrest.
m eine ganze Zahl 0.1 oder 2. wobei für m gleich 2 die beiden Reste C gleich oder verschieden sein können.
n und o gleich oder verschieden jeweils ganze Zahlen 2, 3 oder 4 und
p eine ganze Zahl von 2 bis 6
bedeuten
sowie deren physiologisch verträgliche Salze,
mit der Maßgabe, daß keine Hydrazinderivate umfaßt sind und daß m nicht 0 sein kann, wenn A eine Gruppe (C=NH)NH₂ bedeutet und B und D gleich oder verschieden eine Bindung oder einen Alkylenrest darstellen,

2. Verbindungen der Formel I 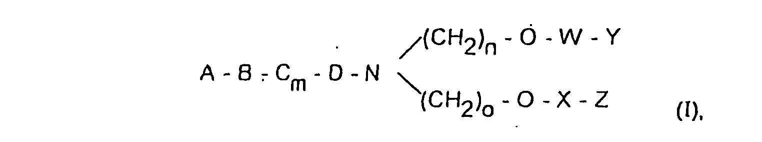 in welcher
A eine Gruppe NR₁R₂, eine Gruppe NR₁(CH₂)ₚNR₃R₄, eine Gruppe (C=NH)NH₂ oder einen Pyridinylrest.
B und D gleich oder verschieden jeweils eine Bindung. einen C₁- bis C₆-Alkylenrest oder eine Gruppe NR₅-C₂- bis C₆-Alkylen.
C Piperidindiyl oder Piperazindiyl.
W und X gleich oder verschieden jeweils eine Bindung oder eine Carbonyleruppe,
Y und Z gleich oder verschieden jeweils einen gesättigten oder ungesättigten Kohlenwasserstoffrest mit 7 bis 24 Köhlenstoffatomen.
R₁ bis R₅ gleich oder verschieden jeweils Wasserstoff oder einen C₁- bis C₆-Alkylrest,
m eine ganze Zahl 1 oder 2, wobei für m gleich 2 die beiden Reste C gleich oder verschieden sein können,
n und o gleich oder verschieden jeweils ganze Zahlen 2. 3 oder 4 und
p eine ganze Zahl von 2 bis 6
bedeuten
sowie deren physiologisch verträgliche Salze,
mit der Maßgabe, daß keine Hydrazinderivate umfaßt sind.

3. Verbindungen nach einem der Ansprüche 1-2, in denen W und X CO bedeuten.

4. Verbindungen nach einem der Ansprüche 1-3, in denen Y C₁₃H₂₇ oder C₁₇ H₃₃ bedeuten.

5. Verbindungen nach einem der Ansprüche 1-4, in denen Z C₁₃H₂₇ oder C₁₇H₃₃ bedeuten.

6. Verbindungen der Formel II 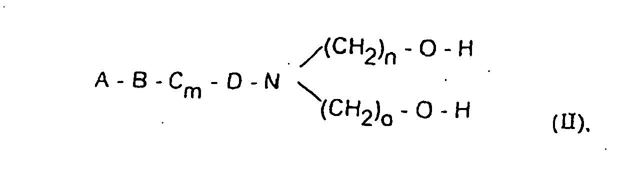 wobei
A eine Gruppe NR₁R₂, eine Gruppe NR₁(CH₂)ₚNR₃R₄, eine Gruppe (C=NH)NH₂ oder einen Pyridinylrest,
B eine Bindung, einen C₁- bis C₆-Alkylenrest oder eine Gruppe NR₅-C₂- bis C₆-Alkylen,
D einen C₁- bis C₆-Alkylenrest oder eine Gruppe NR₅-C₂- bis C₆-Alkylen,
R₁-R₅ gleich oder verschieden jeweils Wasserstoff oder C₁- bis C₆-Alkylenrest bedeuten und
C, n und o die Bedeutungen wie in Anspruch 1 haben und m = 1 oder 2 bedeutet, wobei für m = 2 die beiden Reste C gleich oder verschieden sein können,
mit der Maßgabe, daß keine Hydrazinderivate umfaßt sind.

7. Verbindungen nach einem der Ansprüche 1-6, in denen A NH₂ oder N(CH₃)₂ bedeutet.

8. Verbindungen nach einem der Ansprüche 1-7, in denen B und D gleich oder verschieden sind.

9. Verbindungen nach einem der Ansprüche 1-8, in denen C Piperidindiyl bedeutet.

10. Verbindungen nach einem der Ansprüche 1-9, in denen m 1 bedeutet.

11. Verbindung nach einem der Ansprüche 1-10, in denen n und o 2 bedeutet.

12. Arzneimittel nach einem der Ansprüche 1, 3-5, 7-11, die außerdem noch eine Nukleinsäure enthalten.

13. Arzneimittel nach einem der Ansprüche 1,3-5, 7-11, die außerdem noch ein Therapeutikum enthalten.

14. Verwendung von Verbindungen der Formel I. 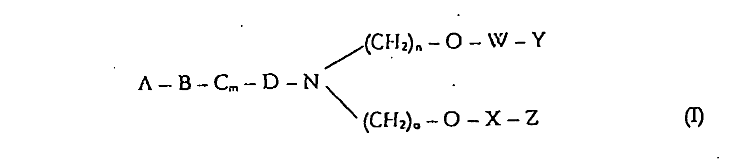 wobei A, B, C, D, W, X, Y, Z, m, n und o die Bedeutung wie in einem der Ansprüche 1-11 haben, mit der Maßgabe, daß keine Hydrazinderivate umfaßt sind zur Herstellung von Genfähren und Transfektionsvehikel mit einem Therapeutikum.

15. Verwendung von Verbindungen gemäß einem der Ansprüche 1-5 und 7-13 zur Herstellung von pharmazeutischen Zusammensetzungen für die Gentherapie.

16. Verwendung von Verbindungen gemäß einem der Ansprüche 1-5 und 7-13 für die in vitro Transfektion.

17. Verwendung von Verbindungen gemäß einem der Ansprüche 1-5 und 7-13 zur Herstellung von in vivo Transfektionsreagenzien.

18. Verwendung von Verbindungen gemäß einem der Ansprüche 1-5 und 7-13 zur Herstellung von Arzneimittelkombination in Krebstherapie, antiviraler Therapie, Infektionstherapie und bei Krankheiten verursacht durch Disregulationen.

## Claims

1. A compound of formula I 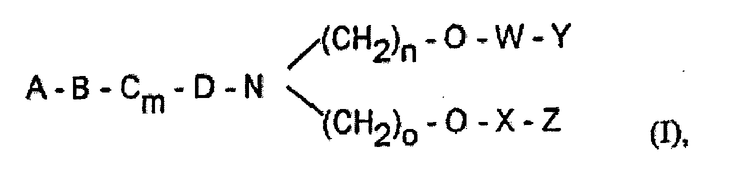 for the use as a pharmaceutical preparation,
in which
A denotes a group NR₁R₂, a group NR₁(CH₂)ₚNR₃R₄, a group (C=NH)NH₂ or a pyridinyl residue,
B and D are the same or different and each denotes a bond, a C₁ to C₆ alkylene residue or a group NR₅-C₂ to C₆ alkylene,
C denotes piperidinediyl or piperazinediyl,
W and X are the same or different and each denotes a bond or a carbonyl group,
Y and Z are the same or different and each denotes a saturated or unsaturated hydrocarbon residue with 7 to 24 carbon atoms,
R₁ to R₅ are the same or different and each represents hydrogen or a C₁ to C₆ alkyl residue,
m is an integer 0, 1 or 2 and if m equals 2 both residues C can be the same or different,
n and o are the same or different and each denotes the integers 2, 3 or 4 and
p denotes an integer from 2 to 6
as well as physiologically tolerated salts thereof,
provided that hydrazine derivatives are not included and that m cannot be 0 if A denotes a group (C=NH)NH₂ and B and D being the same or different represent a bond or an alkylene residue.

2. A compound of formula I 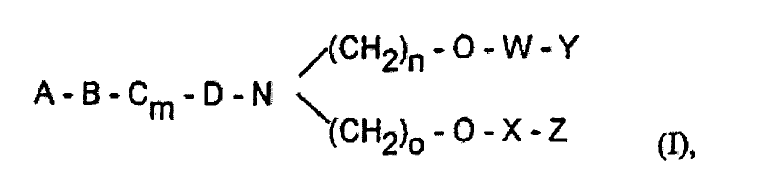 in which
A denotes a group NR₁R₂, a group NR₁(CH₂)ₚNR₃R₄, a group (C-NH)NH₂ or a pyridinyl residue,
B and D are the same or different and each denotes a bond, a C₁ to C₆ alkylene residue or a group NR₅-C₂ to C₆ alkylene,
C denotes piperidinediyl or piperazinediyl,
W and X are the same or different and each denotes a bond or a carbonyl group,
Y and Z are the same or different and each denotes a saturated or unsaturated hydrocarbon residue with 7 to 24 carbon atoms,
R1 to R5 are the same or different and each represents hydrogen or a C₁ to C₆ alkyl residue,
m is an integer 1 or 2 and if m equals 2 both residues C can be the same or different,
n and o are the same or different and each denotes the integers 2, 3 or 4 and
p denotes an integer from 2 to 6
as well as physiologically tolerated salts thereof,
provided that no hydrazine derivatives are not included.

3. Compounds according to claims 1-2, wherein W and X denote CO.

4. Compounds according to claims 1-3, wherein Y denotes C₁₃H₂₇ or C₁₇H₃₃.

5. Compounds according to claims 1-4, wherein Z denotes C₁₃H₂₇ or C₁₇H₃₃.

6. Compounds of the formula II 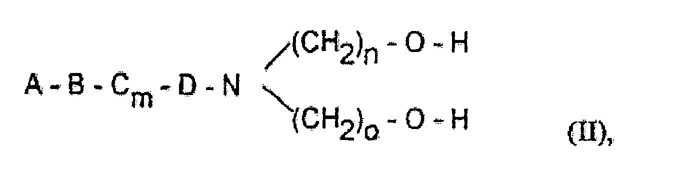 in which A denotes a group NR₁R₂, a group NR₁(CH₂)ₚNR₃R₄, a group (C-NH)NH₂ or a pyridinyl residue,
B denotes a bond, a C₁ to C₆ alkylene residue or a group NR₅-C₂ to C₆ alkylene,
B denotes a C₁ to C₆ alkylene residue or a group NR₅-C₂ to C₆ alkylene,
R1 to R5 are the same or different and each represents hydrogen or a C₁ to C₆ alkyl residue, and
C, n, and o have the same meaning as in claim 1 and m denotes 1 or 2 and if m equals 2 both residues C can be the same or different,
provided that no hydrazine derivatives are not included.

7. Compounds according to claims 1-6, wherein A denotes NH₂ or C(CH₃)₂.

8. Compounds according to claims 1-7, wherein B and D are equal or different.

9. Compounds according to claims 1-8, wherein C denotes piperidinediyl.

10. Compounds according to claims 1-9, wherein m denotes 1.

11. Compounds according to claims 1-10, wherein n and o denote 2.

12. Pharmaceutical preparation according to claims 1, 3-5, 7-11, which additionally comprise a nucleic acid.

13. Pharmaceutical preparation according to claims 1, 3-5, 7-11, which additionally comprise a therapeutically active compound.

14. The use of compound of formula I 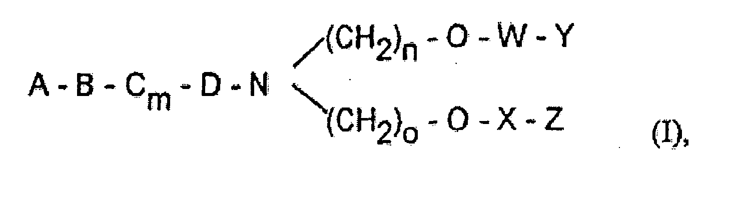 wherein A, B, C, D, W, X, Y, Z, m, n, and o have the same meaning as in claims 1-11, provided that no hydrazine derivatives are not included, for the preparation gene ferries or transfer reagents comprising a therapeutically active compound.

15. The use of compounds according to claims 1-5 and 7-13 for the manufacture of a pharmaceutical composition for gene therapy.

16. The use of compounds according to claims 1-5 and 7-13 for in vitro transfection.

17. The use of compounds according to claims 1-5 and 7-13 for the manufacture of in vivo transfection reagents.

18. The use of compounds according to claims 1-5 and 7-13 for the manufacture of drug combinations in cancer therapy, antiviral therapy, infection therapy and in diseases caused by dysregulation.

## Revendications

1. Composés de formule I, 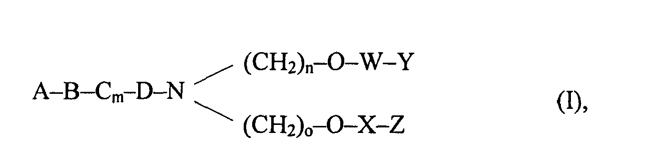 destinés à être utilisés comme médicaments, où
A représente un groupe NR₁R₂, un groupe NR₁(CH₂)ₚNR₃R₄, un groupe (C=NH)NH₂ ou un résidu pyridinyle,
B et D, identiques ou différents, représentent chacun une liaison, un résidu alkylène en C₁ à C₄ ou un groupe NR₅-alkylène en C₂ à C₆,
C représente un résidu pipéridinediyle ou pipérazinediyle,
W et X, identiques ou différents, représentent chacun une liaison ou un groupe carbonyle,
Y et Z, identiques ou différents, représentent chacun un résidu hydrocarboné saturé ou insaturé de 7 à 24 atomes de carbone,
R₁ à R₅, identiques ou différents, représentent chacun un atome d'hydrogène ou un résidu alkyle en C₁ à C₆,
m est un nombre entier valant 0, 1 ou 2, les deux résidus C pouvant être identiques ou différents lorsque m est égal à 2,
n et o, identiques ou différents, sont chacun un nombre entier valant 2, 3 ou 4, et
p est un nombre entier valant de 2 à 6,
ainsi que leurs sels physiologiquement acceptables,
à la condition qu'ils n'englobent pas de dérivés d'hydrazine et que m ne puisse pas être égal à 0 lorsque A représente un groupe (C=NH)NH₂ et lorsque B et D, identiques ou différents, représentent une liaison ou un résidu alkylène.

2. Composés de formule I, 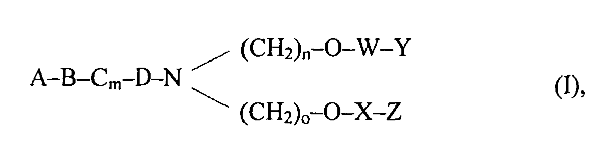 où
A représente un groupe NR₁R₂, un groupe NR₁(CH₂)ₚNR₃R₄, un groupe (C=NH)NH₂ ou un résidu pyridinyle,
B et D, identiques ou différents, représentent chacun une liaison, un résidu alkylène en C₁ à C₄ ou un groupe NR₅-alkylène en C₂ à C₆,
C représente un résidu pipéridinediyle ou pipérazinediyle,
W et X, identiques ou différents, représentent chacun une liaison ou un groupe carbonyle,
Y et Z, identiques ou différents, représentent chacun un résidu hydrocarboné saturé ou insaturé de 7 à 24 atomes de carbone,
R₁ à R₅, identiques ou différents, représentent chacun un atome d'hydrogène ou un résidu alkyle en C₁ à C₆,
m est un nombre entier valant 1 ou 2, les deux résidus C pouvant être identiques ou différents lorsque m est égal à 2,
n et o, identiques ou différents, sont chacun un nombre entier valant 2, 3 ou 4, et
p est un nombre entier valant de 2 à 6,
ainsi que leurs sels physiologiquement acceptables,
à la condition qu'ils n'englobent pas de dérivés d'hydrazine.

3. Composés selon l'une des revendications 1 et 2, dans lesquels W et X représentent CO.

4. Composés selon l'une des revendications 1 à 3, dans lesquels Y représente C₁₃H₂₇ ou C₁₇ H₃₃.

5. Composés selon l'une des revendications 1 à 4, dans lesquels Z représente C₁₃H₂₇ ou C_{I7}H₃₃.

6. Composés de formule II 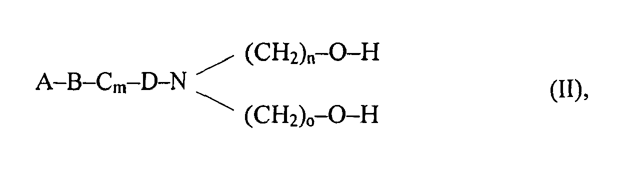 où
A représente un groupe NR₁R₂, un groupe NR₁(CH₂)ₚNR₃R₄, un groupe (C=NH)NH₂ ou un résidu pyridinyle,
B représente une liaison, un résidu alkylène en C₁ à C₆ ou un groupe NR₅-alkylène en C₂ à C₆,
D représente un résidu alkylène en C₁ à C₆ ou un groupe NR₅-alkylène en C₂ à C₆, R₁ à R₅, identiques ou différents, représentent chacun un atome d'hydrogène ou un résidu alkylène en C₁ à C₆, et
C, n et o ont les significations indiquées dans la revendication 1 et m vaut 1 ou 2, les deux résidus C pouvant être identiques ou différents lorsque m est égal à 2,
à la condition qu'ils n'englobent pas de dérivés d'hydrazine.

7. Composés selon l'une des revendications 1 à 6, dans lesquels A représente NH₂ ou N(CH₃)₂.

8. Composés selon l'une des revendications 1 à 7, dans lesquels B et D sont identiques ou différents.

9. Composés selon l'une des revendications 1 à 8, dans lesquels C représente un résidu pipéridinediyle.

10. Composés selon l'une des revendications 1 à 9, dans lesquels m vaut 1.

11. Composés selon l'une des revendications 1 à 10, dans lesquels n et o valent 2.

12. Médicament selon l'une des revendications 1, 3 à 5, 7 à 11, contenant en plus un acide nucléique.

13. Médicament selon l'une des revendications 1, 3 à 5, 7 à 11, contenant en plus un agent thérapeutique.

14. Utilisation de composés de formule I, 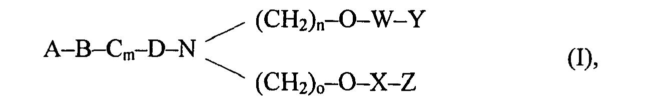 où A, B, C, D, W, X, Y, Z, m, n et o ont les significations indiquées dans l'une des revendications 1 à 11, à la condition qu'ils n'englobent pas de dérivés d'hydrazine, pour la production de vecteurs géniques et de véhicules de transfection avec un agent thérapeutique.

15. Utilisation de composés selon l'une des revendications 1 à 5 et 7 à 13 pour la production de compositions pharmaceutiques destinées à la thérapie génique.

16. Utilisation de composés selon l'une revendications 1 à 5 et 7 à 13 pour la transfection *in vitro.*

17. Utilisation de composés selon l'une des revendications 1 à 5 et 7 à 13 pour la production de réactifs de transfection *in vivo.*

18. Utilisation de composés selon l'une des revendications 1 à 5 et 7 à 13 pour la production d'une combinaison médicamenteuse dans la thérapie anticancéreuse, la thérapie antivirale, la thérapie anti-infectieuse et la lutte contre des maladies résultant de dysrégulations.
